# EUROPEAN PATENT SPECIFICATION

(11) **EP 1 858 852 B1**
(45) Date of publication and mention of the grant of the patent: **01.07.2009**
(21) Application number: 06727159.3
(22) Date of filing: 02.03.2006
(51) Int. Cl.: C07D 213/24, C07D 213/70, C07D 213/73, C07D 213/79, C07D 333/34, A61K 31/44, A61P 25/00

(54) **HETEROARYLSULFONYL STILBENES AS 5-HT2A ANTAGONISTS**
HETEROARYLSULFONYLSTILBENE ALS 5-HT2A-ANTAGONISTEN
HETEROARYLSULFONYLSTILBENES EN TANT QU'ANTAGONISTES DE 5-HT2A

(30) Priority: 09.03.2005 GB 0504828
(43) Date of publication of application: 28.11.2007
(73) Proprietor: MERCK SHARP & DOHME LTD., Hoddesdon, Hertfordshire EN11 9BU (GB)
(72) Inventor: GILLIGAN, Myra, Harlow Essex CM20 2QR (GB); HUMPHRIES, Alexander Charles, Harlow Essex CM20 2QR (GB); LADDUWAHETTY, Tamara, Harlow Essex CM20 2QR (GB); MERCHANT, Kevin John, Harlow Essex CM20 2QR (GB)
(74) Representative: Buchan, Gavin MacNicol
(86) International application number: PCT/GB2006/050044
(87) International publication number: WO 2006/095205

(56) References cited:
- WO-A-86/02927
- US-A- 5 859 257
- US-A1- 2003 130 287
- US-A1- 2003 181 464
- US-B1- 6 559 166
- DATABASE REGISTRY CHEMICAL ABSTRACTS SERVICE, COLUMBUS, OHIO, US; 13 June 2001 (2001-06-13), XP002384978 retrieved from CH

## Description

The present invention relates to a class of sulphonyl derivatives which act on serotonin receptors (also known as 5-hydroxytryptamine or 5-HT receptors). More particularly, the invention concerns heteroarylsulphonylstilbenes and derivatives thereof. These compounds are potent and selective antagonists of the human 5-HT_{2A} receptor and are therefore useful as pharmaceutical agents, especially in the treatment and/or prevention of adverse conditions of the central nervous system, including sleep disorders such as insomnia, psychotic disorders such as schizophrenia and psychiatric disorders such as anxiety.

Compounds of the invention typically display more effective binding to the human 5-HT_{2A} receptor than to other human receptors such as D₂, 5HT_{2C} and IKr receptors. They can therefore be expected to manifest fewer side-effects than compounds which do not discriminate in their binding affinity between such receptors. In particular these compounds have lower effects on the IKr receptors and there is a separation of the desired effect from side effects such as cardiac effects.

By virtue of their potent human 5-HT_{2A} receptor antagonist activity, the compounds of the present invention are effective in the treatment of neurological conditions including sleep disorders such as insomnia, psychotic disorders such as schizophrenia, and also depression, anxiety, panic disorder, obsessive-compulsive disorder, pain, eating disorders such as anorexia nervosa, and dependency or acute toxicity associated with narcotic agents such as LSD or MDMA; and moreover are beneficial in controlling the extrapyramidal symptoms associated with the administration of neuroleptic agents. They are also effective in the lowering of intraocular pressure, and hence in the treatment of glaucoma, and may also be effective in treating menopausal symptoms, in particular hot flushes (see Waldinger et al, Maturitas, 2000, 36, 165-8).

Various classes of compounds containing *inter alia* a sulphonyl moiety are described in WO 2005/047246, WO 2005/047247, WO 03/099786 WO 2004/101518, WO 01/74797, WO 00/43362, WO 96/35666, EP-A-0261688, EP-0304888, and US Patents 4,218,455 and 4,128,552, DE-A-3901735 and Fletcher et al, J. Med. Chem., 2002, 45, 492-503. None of these publications, however, discloses or suggests the particular class of compounds provided by the present invention.

The compounds according to the present invention are potent and selective 5-HT_{2A} receptor antagonists, suitably having a human 5-HT_{2A} receptor binding affinity (Kᵢ) of 100 nM or less, typically of 50 nM or less and preferably of 10 nM or less. The compounds of the invention may possess at least a 10-fold selective affinity, suitably at least a 20-fold selective affinity and preferably at least a 50-fold selective affinity, for the human 5-HT_{2A} receptor relative to the human dopamine D₂ receptor and/or the human IKr and/or 5-HT_{2c} receptors. Preferred compounds show selectivities of at least 100-fold relative to the human 5-HT_{2c} receptor.

The present invention provides a compound of formula I: 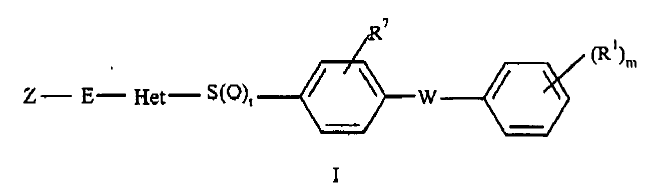 or a pharmaceutically acceptable salt thereof; wherein:
m is 0, 1, 2 or 3;
t is 1 or 2;
Het represents a pyridine or thiophene ring bearing 0, 1 or 2 R² substituents;
W represents -CR³R⁴-CR⁵R⁶, -CR³=CR⁵- or -C=C- where R³, R⁴, R⁵, and R⁶ are selected from H, OH and F but not more than one of R³, R⁴, R⁵, and R⁶ is other than H; or R³ and R⁴ together or R⁵ and R⁶ together complete a keto group; or R⁴ and R⁶ together complete a cyclopropyl ring;
E represents a chemical bond or a straight or branched alkylene chain containing from 1 to 4 carbon atoms, optionally incorporating an oxygen atom to form an ether linkage;
Z is selected from halogen, CN, nitro, CF₃, OCF₃, -R^{a}, -OR^{a}, -SR^{a}, -SOR^{a}, -SO₂R^{a}, -SO₂NR^{a}R^{b},-NR^{a}R^{b}, -NR^{a}COR^{b}, -NR^{a}CO₂R^{b}, -NR^{a}CO₂NR^{a}R^{b}, -NR^{a}S(O)ₜR^{a}, -NF^{a}SO₂NR^{a}R^{b}, -COR^{a}, -CO₂R^{a}, -CONR^{a}R^{b}, -CH=NOR^{a} or a five- or six-membered heteroaromatic ring optionally bearing up to 2 substituent selected from halogen, CN, CF₃, C₁₋₆alkyl, C₁₋₆alkoxy, C₁₋₆alkylthio, amino, C₁₋₆alkylamino and di(C₁₋₆)alkylamino;
R^{a} and R^{b} independently represent H or a hydrocarbon group of up to 7 carbon atoms which is optionally substituted with up to 3 fluorine atoms and optionally with Cl, Br, CN, OH, C₁₋₄alkoxy, C₁₋₄ alkylthio, amino, C₁₋₄alkylamino or di(C₁₋₄)alkylamino; or R^{a} and R^{b}, when linked through a nitrogen atom, together represent the residue of a heterocyclic ring of 4, 5 or 6 members, optionally bearing up to 3 substituents selected from halogen, CN, CF₃, oxo, OH, C₁₋₄alkyl and C₁₋₄alkoxy;
each R¹ independently represents halogen, CN, CF₃, OCF₃, C₁₋₆ alkyl, OH, benzylthio, C₁₋₆ alkoxy or hydroxymethyl;
each R² independently represents halogen, CN, CONH₂, C₁₋₄alkyl or C₁₋₄alkoxy;
and R⁷ represent H, halogen, CN, CF₃, OR^{a}, CO₂R^{a}, CONR^{a}R^{b}, NR^{a}R^{b} or C₁₋₄alkyl which is optionally substituted with halogen, CN, CF₃, OR^{a}, CO₂R^{a}, CONR^{a}R^{b} or NR^{a}R^{b}.

In a particular embodiment, the invention provides a compound of formula IA: 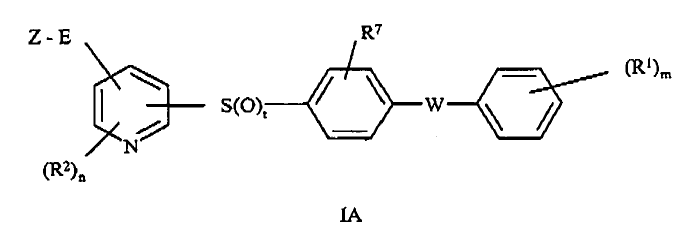 or a pharmaceutically acceptable salt thereof; wherein:
n is 0, 1 or 2;
and m, t, W, E, Z, R¹, R² and R⁷ are as defined previously.

Where a variable occurs more than once in formula I or in a substituent group thereof, the individual occurrences of that variable are independent of each other, unless otherwise specified.

As used herein, the expression "hydrocarbon goup" refers to groups consisting solely of carbon and hydrogen atoms. Such groups may comprise linear, branched or cyclic structures, singly or in any combination consistent with the indicated maximum number of carbon atoms, and may be saturated or unsaturated, including aromatic when the indicated maximum number of carbon atoms so permits unless otherwise indicated.

As used herein, the expression "C₁₋ₓalkyl" where x is an integer greater than 1 refers to straight-chained and branched alkyl groups wherein the number of constituent carbon atoms is in the range 1 to x. Particular alkyl groups are methyl, ethyl, n-propyl, isopropyl and t-butyl. Derived expressions such as "C₂₋₆alkenyl", "hydroxyC₁₋₆alkyl", "heteroarylC₁₋₆alkyl", "C₂₋₆alkynyl" and "C₁₋₆alkoxy" are to be construed in an analogous manner. Most suitably, the number of carbon atoms in such groups is not more than 6.

The term "halogen" as used herein includes fluorine, chlorine, bromine and iodine, of which fluorine and chlorine are preferred and fluorine particularly preferred.

The expression "C₃₋₆cycloalkyl" as used herein refers to nonaromatic monocyclic hydrocarbon ring systems comprising from 3 to 6 ring atoms. Examples include cyclopropyl, cyclobutyl, cyclopentyl, cyclohexyl and cyclohexenyl.

For use in medicine, the compounds of formula I may be in the form of pharmaceutically acceptable salts. Other salts may, however, be useful in the preparation of the compounds of formula I or of their pharmaceutically acceptable salts. Suitable pharmaceutically acceptable salts of the compounds of this invention include acid addition salts which may, for example, be formed by mixing a solution of the compound according to the invention with a solution of a pharmaceutically acceptable acid such as hydrochloric acid, sulphuric acid, methanesulphonic acid, benzenesulphonic acid, fumaric acid, maleic acid, succinic acid, acetic acid, benzoic acid, oxalic acid, citric acid, tartaric acid, carbonic acid or phosphoric acid. Alternatively, where the compound of the invention carries an acidic moiety, a pharmaceutically acceptable salt may be formed by neutralisation of said acidic moiety with a suitable base. Examples of pharmaceutically acceptable salts thus formed include alkali metal salts such as sodium or potassium salts; ammonium salts; alkaline earth metal salts such as calcium or magnesium salts; and salts formed with suitable organic bases, such as amine salts (including pyridinium salts) and quaternary ammonium salts.

When the compounds according to the invention have one or more asymmetric centres, they may accordingly exist as enantiomers. Where the compounds according to the invention possess two or more asymmetric centres, they may additionally exist as diastereoisomers. It is to be understood that all such isomers and mixtures thereof in any proportion are encompassed within the scope of the present invention.

In formula I, the heteroaryl ring represented by Hct is selected from pyridine and thiophene. In another embodiment, Het represents pyridine and the compounds are in accordance with formula IA.

In the compounds of formula 1, t is 1 or 2. In a particular embodiment, t is 2.

The moiety S(O)ₜ may be attached at any of the available positions on the ring represented by Het. When Het represents a pyridine ring, S(O)ₜ is very suitably attached to the 2- or 3-position thereof. When Het represents a thiophene ring, S(O)ₜ is very suitably attached to the 2-position thereof.

W represents -CR³R⁴-CR⁵R⁶-, -CR³=CR⁵- or -C≡C- where R³, R⁴, R⁵, and R⁶ are as defined previously. Suitable identities of W include -CH₂CH₂-, -CHFCH₂-, -CH₂CHF-, -CH(OH)CH₂-, -CH₂CH(OH)-, -COCH₂-, -CH₂CO-, -CH=CH-, -C=C-, -CF=CH-, -CH=CF-, -C(OH)=CH-, -CH=C(OH)- and cyclopropane-1,2-diyl. It will be readily apparent that compounds of formula I in which W is -COCH₂- or -CH₂CO- are tautomeric with the corresponding compounds of formula I in which W is (respectively) -C(OH)=CH- or -CH=C(OH)-. Both forms, singly or in mixtures of any proportion, are within the scope of the invention. In one preferred embodiment, W represents -CH₂CH₂-. In another preferred embodiment, W represents -CH=CH-.

Where E represents a straight or branched alkylene chain, this may be, for example, methylene, ethylene, 1-methylethylene, propylene, 2-methylpropylene or butylene. The alkylene chain E may optionally incorporate an oxygen atom, thereby forming an ether linkage such as -CH₂O- or -CH₂CH₂CH₂O-. Moreover, E may represent a chemical bond such that the moiety Z is attached directly to the relevant phenyl ring depicted in formula 1 above.

Preferably, E represents a chemical bond or a methylene linkage.

In a specific embodiment, E represents a chemical bond.

In another specific embodiment, E represents a methylene linkage.

Z preferably represents halogen, CN, CF₃, R^{a}, OR^{a}, SR^{a}, SO₂R^{a}, SO₂NR^{a}R^{b}, NR^{a}R^{b}, NR^{a}COR^{b}, NR^{a}CONR^{a}R^{b}, NR^{a}SOR^{a}, NR^{a}SO₂R^{a}, COR^{a}, CO₂R^{a}, CONR^{a}R^{b}, CH=NOR^{a} or a five- or six-membered heteroaromatic ring optionally bearing up to 2 substituents as defined previously.

Where the group Z represents an optionally substituted five-membered heteroaromatic ring, this is suitably an imidazole, pyrazole, 1,2,3-triazole, 1,2,4-triazole or tetrazole ring, any of which optionally is substituted, typically by methyl. Such rings may be attached via a carbon atom or a nitrogen atom. Specific examples include pyrazol-1-yl, imidazol-1-yl and 2-methyl-1,2,4-triazol-3-yl.

Where the group Z represents an optionally substituted six-membered heteroaromatic ring, this is suitably a pyridine, pyrazine, pyrimidine, pyridazinc or triazine ring, any of which optionally is substituted, typically by methyl or halogen. A specific example is 2-pyridyl.

R^{a} and R^{b} independently represent H or an optionally substituted hydrocarbon group as defined previously, or when linked through a nitrogen atom they may complete an optionally-substituted heterocyclic ring as defined previously. Hydrocarbon groups represented by R^{a} or R^{b} are preferably nonaromatic. Said hydrocarbon groups optionally bear up to 3 fluorine substituents and, in addition or as an alternative, optionally bear a substituent selected from Cl, Br, CN, OH, C₁₋₄alkoxy, C₁₋₄alkylthio, amino, C₁₋₄alkylamino and di(C₁₋₄alkyl)amino. Preferred substituents include F, OH and CN. Typically, R^{a} and R^{b} independently represent H; optionally substituted C₁₋₆alkyl (such as methyl, ethyl, isopropyl, tert-butyl, 2,2,2-trifluoroethyl, 2-cyanoethyl, hydroxymethyl, 1-hydroxyethyl, 2-hydroxyethyl, 1-hydroxypropyl, 1-hydroxy-1-methylethyl and 1-hydroxy-2,2,2-trifluoroethyl); optionally substituted C₃₋₆cycloalkyl (such as cyclopropyl, cyclobutyl and 1-hydroxycyclobutyl); C₃₋₆cycloalkylC₁₋₄alkyl (such as cyclopropylmethyl); or, when linked through a nitrogen atom, together represent the residue of a heterocyclic ring of 4, 5 or 6 members optionally bearing up to 3 substituents as defined previously. Such rings typically comprise at most two heteroatoms selected from N, O and S, inclusive of the nitrogen atom connecting R^{a} and R^{b}, for example azetidine, pyrrolidine, piperidine, tetrahydropyridine, piperazine, morpholine and thiomorpholine. Typical examples of cyclic groups represented by NR^{a}R^{b} include azetidin-1yl, 3,3-difluoroazetidin-1-yl, 3-hydroxyazetidin-1-yl, pyrrolidin-1-yl, 3-hydroxypyrrolidin-1-yl, 3-fluoropyrrolidin-1-yl, 2-trifluoromethylpyrrolidin-1-yl, piperidin-1-yl, 4-trifluoromethylpiperidin-1-yl, 3-trifluoromethylpiperidin-1-yl, 3-fluoropiperidin-1-yl, 3,3,-difluoropiperidin-1-yl, 4,4-difluoropiperidin-1-yl, 4-trifluoromethyl-1,2,3,6-tetrahydropyridin-1-yl, 4-methylpiperazin-1-yl, 3-oxo-piperazin-1-yl, morpholin-4-yl, 2,6-dimethylmorpholin-4-yl and 1,1-dioxo-thiomorpholin-4-yl.

When Z represents R^{a}, R^{a} very suitably represents H or optionally-substituted C₁₋₆alkyl or optionally substituted C₃₋₆cycloalkyl and E suitably represents a chemical bond.

Preferred identities for the moiety -E-Z include H, isopropyl, 2-cyanoethyl, hydroxymethyl, 1-hydroxyethyl, 2-hydroxyethyl, 1-hydroxypropyl, 1-hydroxy-1-methylethyl, 1-hydroxy-2,2,2-trifluoroethyl, 1-hydroxycyclobutyl, CO₂Me, CO₂Et, CONH₂, CONHMe, COCH₃, NH₂, NHMe, NMe₂, NHSO₂Me, SO₂Me, CN, CH₂NH₂, CH₂NHSO^{t-}Bu, CH₂NHCOMe, morpholin-4-yl and morpholin-4-ylmethyl.

The heteroaryl ring to which the moiety -E-Z is attached optionally bears up to two additional substituents R² as defined previously. Typically, n is 0 or 1 and hence not more than one R² group is present. Most preferably, n is 0. When present, preferred identities for R² include halogen (especially F), C₁₋₄alkyl (especially methyl) CN and CONH₂.

In formula I, m represents 0,1,2 or 3, but preferably represents 1 or 2. Each R¹ is preferably selected from halogen (preferably F or Cl, most preferably F), CN, C₁₋₄alkyl (especially methyl), hydroxymethyl, OH and C₁₋₄alkoxy (e.g. methoxy). Specific embodiments of (R¹)ₘ include H, 2-fluoro, 3-fluoro, 4-fluoro, 2,4-difluoro, 3-cyano, 4-cyano, 2-chloro-4-fluoro, 4-fluoro-2-methyl, 4-fluoro-2-hydroxy, 4-chloro, 2-hydroxy, 2-cyano-4-fluoro, 4-fluoro-2-methoxy, 4-fluoro-2-hydroxymethyl and 2-methyl. In a particular embodiment, (R¹)ₘ represents 4-fluoro or 2,4-difluoro substitution of the phenyl ring.

R⁷ preferably represents H, halogen (such as Br or Cl), CN or CONH₂. Most preferably, R⁷ represents H. In a particular embodiment, the invention provides a compound of formula II: 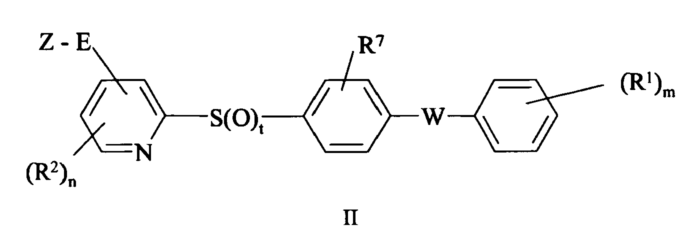 or a pharmaceutically acceptable salt thereof;
where all the variables have the same meanings and preferred identities as before.

Within this embodiment, the moiety Z-E- is preferably attached at a ring position which is adjacent to the point of attachment of the -S(O)ₜ- moiety or adjacent to the ring nitrogen.

In another particular embodiment, the invention provides a compound of formula III: 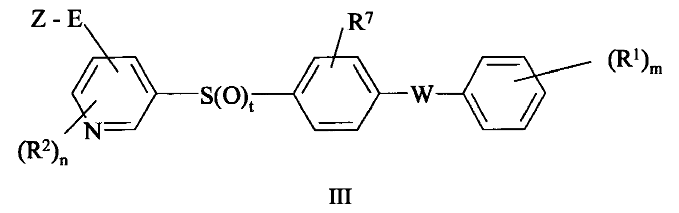 or a pharmaceutically acceptable salt thereof;
where all the variables have the same meanings and preferred identities as before.

Within this embodiment, the moiety Z-E- is preferably attached at a ring position which is adjacent to the point of attachment of the -S(O)ₜ- moiety and/or adjacent to the ring nitrogen.

In a particular embodiment, the invention provides a compound of formula IV: 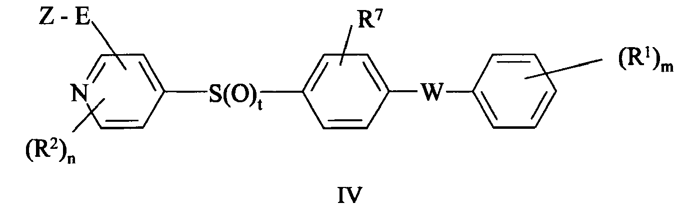 or a pharmaceutically acceptable salt thereof;
where all the variables have the same meanings and preferred identities as before.

Specific compounds of this invention include those compounds exemplified hereinafter and their pharmaceutically acceptable salts.

The compounds of the present invention have an activity as antagonists of the human 5-HT_{2A} receptor and hence find use in the treatment or prevention of disorders mediated by 5-HT_{2A} receptor activity.

The invention also provides pharmaceutical compositions comprising one or more compounds of this invention and a pharmaceutically acceptable carrier. Preferably these compositions are in unit dosage forms such as tablets, pills, capsules, powders, granules, sterile parenteral solutions or suspensions, metered aerosol or liquid sprays, drops, ampoules, transdermal patches, auto-injector devices or suppositories; for oral, parenteral, intranasal, sublingual or rectal administration, or for administration by inhalation or insufflation. The principal active ingredient typically is mixed with a pharmaceutical carrier, e.g. conventional tableting ingredients such as corn starch, lactose, sucrose, sorbitol, talc, stearic acid, magnesium stearate and dicalcium phosphate, or gums, dispersing agents, suspending agents or surfactants such as sorbitan monooleate and polyethylene glycol, and other pharmaceutical diluents, e.g. water, to form a homogeneous preformulation composition containing a compound of the present invention, or a pharmaceutically acceptable salt thereof. When referring to these preformulation compositions as homogeneous, it is meant that the active ingredient is dispersed evenly throughout the composition so that the composition may be readily subdivided into equally effective unit dosage forms such as tablets, pills and capsules. This preformulation composition is then subdivided into unit dosage forms of the type described above containing from 0.1 to about 500 mg of the active ingredient of the present invention. Typical unit dosage forms contain from 1 to 100 mg, for example 1, 2, 5, 10, 25, 50 or 100 mg, of the active ingredient. Tablets or pills of the novel composition can be coated or otherwise compounded to provide a dosage form affording the advantage of prolonged action. For example, the tablet or pill can comprise an inner dosage and an outer dosage component, the latter being in the form of an envelope over the former. The two components can be separated by an enteric layer which serves to resist disintegration in the stomach and permits the inner component to pass intact into the duodenum or to be delayed in release. A variety of materials can be used for such enteric layers or coatings, such materials including a number of polymeric acids and mixtures of polymeric acids with such materials as shellac, cetyl alcohol and cellulose acetate.

The liquid forms in which the novel compositions of the present invention may be incorporated for administration orally or by injection include aqueous solutions, liquid- or gel-filled capsules, suitably flavoured syrups, aqueous or oil suspensions, and flavoured emulsions with edible oils such as cottonseed oil, sesame oil or coconut oil, as well as elixirs and similar pharmaceutical vehicles. Suitable dispersing or suspending agents for aqueous suspensions include synthetic and natural gums such as tragacanth, acacia, alginate, dextran, sodium carboxymethylcellulose, methylcellulose, poly(ethylene glycol), poly(vinylpyrrolidone) or gelatin.

The present invention also provides a compound of formula I or a pharmaceutically acceptable salt thereof for use in a method of treatment of the human body. Preferably the treatment is for a condition mediated by 5-HT_{2A} receptor activity.

The present invention further provides the use of a compound of formula I or a pharmaceutically acceptable salt thereof in the manufacture of a medicament for treating or preventing a condition mediated by 5-HT_{2A} receptor activity.

Also disclosed is a method of treatment of a subject suffering from or prone to a condition mediated by 5-HT_{2A} receptor activity which comprises administering to that subject an effective amount of a compound according to formula I or a pharmaceutically acceptable salt thereof.

In one aspect of the invention, the condition mediated by 5-HT_{2A} receptor activity is sleep disorder, in particular insomnia. In a further aspect of the invention, the condition mediated by 5-HT_{2A} receptor activity is selected from psychotic disorders (such as schizophrenia), depression, anxiety, panic disorder, obsessive-compulsive disorder, pain, glaucoma, eating disorders (such as anorexia nervosa), dependency or acute toxicity associated with narcotic agents such as LSD or MDMA, and hot flushes associated with the menopause.

In the treatment envisaged herein, for example of insomnia or schizophrenia, a suitable dosage level is about 0.01 to 250 mg/kg per day, preferably about 0.05 to 100 mg/kg per day, and especially about 0.05 to 5 mg/kg per day. The compounds may be administered on a regimen of 1 to 4 times per day but preferably once per day, for example before going to bed.

If desired, the compounds according to this invention may be co-administered with another sleep inducing or anti-schizophrenic or anxiolytic medicament. Such co-administration may be desirable where a patient is already established on sleep inducing or anti-schizophrenic or anxiolytic treatment regime involving other conventional medicaments. In particular, for the treatment of sleep disorders, the compounds of the invention may be co-administered with a GABA_{A} receptor agonist such as gaboxadol, or with a short term and/or rapid-onset hypnotic such as zolpidem, or a benzodiazepine, a barbiturate, a prokineticin modulator, an antihistamine, trazodone, or derivative of trazodone as disclosed in WO 03/068148.

According to a further aspect of the invention, there is provided the combination of a compound of formula I or a pharmaceutically acceptable salt or hydrate thereof and gaboxadol for use in treatment or prevention of sleep disorders, schizophrenia or depression.

Also according to the invention, there is provided a method of treatment or prevention of sleep disorders, schizophrenia or depression comprising administering to a subject in need thereof a compound of formula I or a pharmaceutically acceptable salt or hydrate thereof in combination with gaboxadol.

As used herein, the expression "in combination with" requires that therapeutically effective amounts of both a compound of formula I or a pharmaceutically acceptable salt or hydrate thereof and gaboxadol are administered to the subject, but places no restriction on the manner in which this is achieved. Thus, the two species may be combined in a single dosage form for simultaneous administration to the subject, or may be provided in separate dosage forms for simultaneous or sequential administration to the subject. Sequential administration may be close in time or remote in time, e.g. one species administered in the morning and the other in the evening. The separate species may be administered at the same frequency or at different frequencies, e.g. one species once a day and the other two or more times a day. The separate species may be administered by the same route or by different routes, e.g. one species orally and the other parenterally, although oral administration of both species is preferred, where possible.

According to a further aspect of the invention there is provided a pharmaceutical composition comprising, in a pharmaceutically acceptable carrier, a compound of formula I or a pharmaceutically acceptable salt or hydrate thereof and gaboxadol.

The invention further provides the use, for the manufacture of a medicament for treatment or prevention of sleep disorders, schizophrenia or depression, of a compound of formula I or a pharmaceutically acceptable salt or hydrate thereof and gaboxadol.

The invention further provides a kit comprising a first medicament comprising a compound of formula I or a pharmaceutically acceptable salt or hydrate thereof and a second medicament comprising gaboxadol together with instructions for administering said medicaments sequentially or simultaneously to a patient suffering from a sleep disorder, schizophrenia or depression.

As used herein, the term "gaboxadol" is inclusive of 4,5,6,7-tetrahydroisoxazolo[5,4-*c*]pyridin-3-ol in free base or zwitterionic form and also of pharmaceutically acceptable acid addition salts thereof such as the hydrochloride salt. Most suitably, gaboxadol is in the form of a crystalline monohydrate of the zwitterionic form.

Compounds of formula I in which W is -CH=CH- may be obtained by reacting a compound of formula (1a) with a styrene of formula (2a): 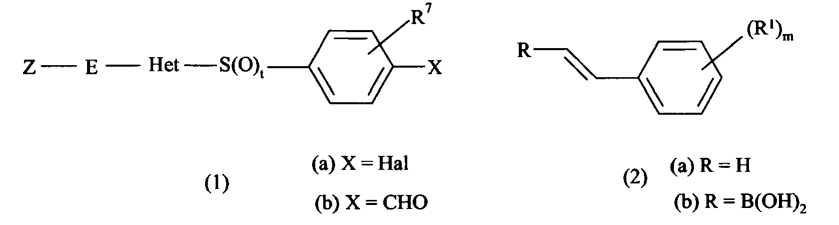 where Hal represents Cl or Br and all other variables have the same meanings as before. The reaction takes place at elevated temperature (e.g. 130°C) in 1-methylpyrrolidone in the presence of palladium acetate and sodium acetate. "Hal" is preferably Br.

Alternatively, the compound of formula (1a) may be reacted with a boronic acid derivative (2b), typically in THF solution in the presence of (Ph)₄Pd[0] and a base such as sodium carbonate with heating (e.g. to 150°C via microwave irradiation).

In a further alternative, an aldehyde of formula (1b) is coupled with a benzylphosphonate such as (3a) or a benzylphosphonium salt such as (3b): 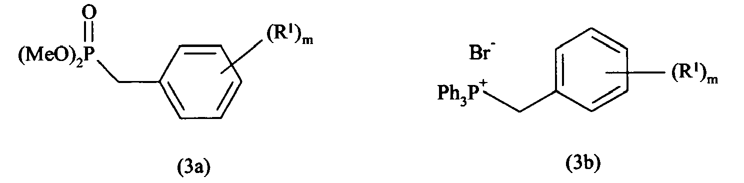 where R¹ and m have the same meanings as before. The reaction may be carried out in THF in the presence of strong base such as BuLi or the combination of sodium hydride with a crown ether.

In a further alternative, a compound of formula (1a) may be treated with tributyl(vinyl)tin to provide an alkene (4) which may be coupled with a bromobenzene (or iodobenzene) (5): 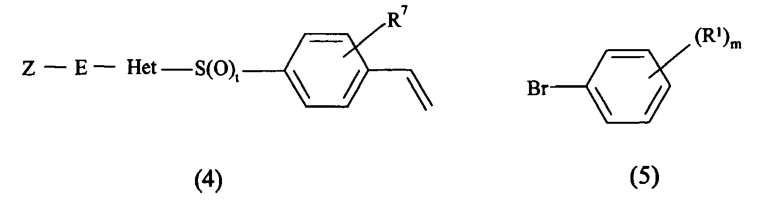 where all variables have the same meanings as before. The coupling takes place under similar conditions to the coupling of (1a) with (2a).

Compounds of formula (1a) and (1b) are obtainable by reaction of compounds (6) with compounds (7) followed by oxidation of the resulting thioether (8): 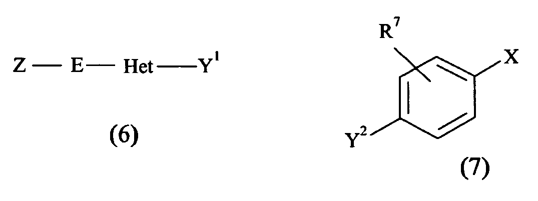 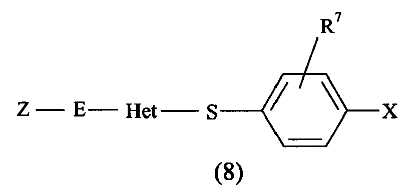 where either Y¹ is I and Y² is SH or Y¹ is SH and Y² is I, and all other variables have the same meanings as before. Formation of thioethers (8) takes place in the presence of CuI and ethylene glycol and a base such as potassium carbonate in a solvent such as isopropanol. Oxidation of thioethers (8) with one equivalent of oxidant (e.g. m-chloroperoxybenzoic acid) provides sulphoxides (1a) in which t = 1. Use of excess oxidant provides sulphones (1a) in which t = 2.

The aforementioned sulphones may also be obtained directly by the reaction between a compound of formula (6) and a compound of formula (7) wherein one of Y¹ and Y² is I or Br and the other is SO₂Na⁺. This reaction may be carried out in DMSO solution at 110°C in the presence of a Cu(I) salt such as the iodide or triflate.

Compounds of formula I in which W is -CH₂CO- or its tautomeric form -CH=C(OH)- are obtainable by reaction of a compound of formula (1a) with an acetophenone (9): 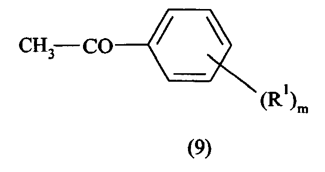 where R¹ and m have the same meaning as before. The reaction may be carried out in refluxing THF under N₂ in the presence of a base such as potassium phosphate under palladium catalysis.

Compounds of formula I in which W is -C≡C- may be obtained by reacting an aldehyde (1b) with diethyl(1-diazo-2-oxopropyl)phosphonate and coupling the resulting alkyne with the appropriate iodobenzene or bromobenzene (5). The first step takes place in the presence of potassium carbonate in an alkanol, and the coupling reaction takes place in the presence of CuI and a Pd(II) catalyst such as (Ph₃P)₂PdCl₂.

Compounds of formula I in which W is -CH(OH)CH₂ may be obtained by reaction of an aldehyde (1b) with the appropriate benzylzinc halide. The reaction may be carried out in THF at -78°C in the presence of a Cu(I) salt and BF₃ etherate.

Compounds of formula I in which W is -CH₂CH₂- may be obtained by hydrogenation of the corresponding compounds in which W is -CH=CH-, e.g. over Pd/C or PtO₂.

It will be readily apparent that the order in which the reaction steps outlined above are carried out may be varied. For example, it is possible to couple a compound of formula (9) or (2a) or (2b) with a compound of formula (7) (X=Hal) and to react the product with a compound of formula (6) under similar conditions to those outlined above.

Where they are not themselves commercially available, the starting materials and reagents described above may be obtained from commercially available precursors by means of well known synthetic procedures and/or the methods disclosed in the Examples section herein.

It will be appreciated that any compound of formula I initially obtained from any of the above processes may, where appropriate, subsequently be elaborated into a further desired compound of formula I using techniques known from the art. For example, a bromo substituent represented by Z-E-, R¹, R² or R⁷ may be replaced by cyano by treatment with copper(I) cyanide in the presence of 1-methyl-2-pyrrolidinone (NMP), or with zinc cyanide in the presence of tetrakis(triphenylphosphine)palladium(0). The cyano group thereby obtained may in turn be converted into carboxamido by heating in mineral acid, e.g. 85% sulphuric acid at 100°C, or by treatment with potassium trimethylsilanolate, typically in tetrahydrofuran at reflux, or by treatment with alkaline hydrogen peroxide. Similarly, a fluoro substituent represented by Z-E- or R⁷ may be replaced by NR^{a}R^{b} or an optionally substituted N-linked heteroaryl moiety, e.g. imidazol-1-yl, pyrazol-1-yl, 1,2,3-triazol-1-yl or 1,2,4-triazol-1-yl, by treatment with HNR^{a}R^{b} or the appropriate optionally substituted N-containing heteroaryl compound, typically with heating in DMSO. Similarly, a bromo substituent represented by Z-E- may be replaced by an optionally substituted C-linked five-membered heteroaromatic ring, e.g. 2-methyltetrazol-5-yl or 1-methyl-1,2,4-triazol-5-yl, by reaction with a tributylstannyl derivative of the appropriate heteroaromatic compound, e.g. 2-methyl-5-tributylstannyltetrazole or 1-methyl-5-tributylstannyl-1,2,4-triazole, in the presence of a transition metal catalyst such as tetrakis(triphenylphosphine)palladium(0), typically with heating in a solvent such as *N,N-*dimethylformamide. A cyano substituent represented by Z-E- may be converted to CHO by diisobutylaluminium hydride (DIBAL-H) reduction and hydrolysis. A CHO substituent represented by Z-E- may be converted to CH₂NR^{a}R^{b} by treatment with HNR^{a}R^{b} and sodium triacetoxyborohydride or sodium cyanoborohydride. A substituent COR^{a} represented by Z-E- may be converted to CH(OH)R^{a} by reduction (e.g. using sodium borohydride) or to CR^{a}(OH)R^{b} by treatment with R^{b}MgHal where Hal is Cl, Br or I. Compounds in which Z-E- take the form Z-(CH₂)_{y}-O- where y is 1, 2, 3, or 4 may be formed by treating the corresponding compounds in which Z-E- is F with Z-(CH₂)_{y}OH in the presence of strong base.

Such processes may also be used to prepare appropriately-substituted precursors of the compounds of Formula I and/or to manipulate the identity of R⁷. A preferred route to compounds (6) wherein Het represents pyridine, Y¹ represents 2-bromo and Z-E- represents 1-hydroxyalkyl or 1-hydroxycycloalkyl attached to the 3-position comprises treatment of 2-bromopyridine with lithium diisopropylamide followed by the appropriate ketone.

Compounds wherein W comprises CO may be reduced to provide corresponding compounds wherein W comprises CH(OH), e.g. using NaBH₄. These in turn may be treated with (diethylamino)sulfur trifluoride to provide compounds wherein W comprises CHF.

Where the above-described processes for the preparation of the compounds of use in the invention give rise to mixtures of stereoisomers, these isomers may be separated by conventional techniques such as preparative chromatography. The compounds may be prepared in racemic form, or individual enantiomers may be prepared either by enantiospecific synthesis or by resolution. The compounds may, for example, be resolved into their component enantiomers by standard techniques such as preparative HPLC, or the formation of diastereomeric pairs by salt formation with an optically active acid, such as di-p-toluoyl-D-tartaric acid and/or di-p-toluoyl-L-tartaric acid, followed by fractional crystallization and regeneration of the free base. The compounds may also be resolved by formation of diastereomeric esters or amides, followed by chromatographic separation and removal of the chiral auxiliary.

During any of the above synthetic sequences it may be necessary and/or desirable to protect sensitive or reactive groups on any of the molecules concerned. This may be achieved by means of conventional protecting groups, such as those described in Protective Groups in Organic Chemistry, ed. J.F.W. McOmie, Plenum Press, 1973; and T.W. Greene & P.G.M. Wuts, Protective Groups in Organic Synthesis, John Wiley & Sons, 1991. The protecting groups may be removed at a convenient subsequent stage using methods known from the art. As an example this protocol, compounds of formula (7) in which Y² is SO₂⁻Na⁺ may be converted to the corresponding 2-(arylsulfonyl)propanenitriles by reaction with acrylonitrile (e.g. in aqueous acetic acid at 100°C), prior to reaction of the group X with a compound of formula (2a), (2b), (3a), (3b) or (9). Thereafter, the sulfinate group may be regenerated by treatment with sodium methoxide (e.g. in a methanol/THF mixture at ambient temperature).
Compounds were tested for their binding to the 5-HT_{2A} receptor and to other receptors such as 5-HT_{2C} and IKr using the methodology described in Fletcher et al, J. Med. Chem., 2002, 45, 492-503.

### EXAMPLES

### Styrylboronic acid intermediates

### [(E)-2-(2,4-difluorophenyl)vinyl]boronic acid

1-Ethynyl-2,4-difluorobenzene (9.6g, 69.5 mmol) was warmed to 40°C and catechol borane (8.3g, 69.2 mmol) was added. The dark reaction mixture was stirred at 40°C for 3 hours before stirring at 80°C for 24 hours. Room temperature was attained and the mixture left to stand for 2 days. Water was added and the resulting dark solid collected by filtration. The solid was washed on the sinter with toluene to leave a beige solid, identified as [(*E*)-2-(2,4-difluorophenyl)vinyl]boronic acid and a mixture of anhydrides (3.8g).

[(*E*)-2-(4-difluorophenyl)vinyl]boronic acid was prepared similarly starting from 1-ethynyl-4-fluorobenzene.

### Example 1

### 2-({4-[(E)-2-(4-fluorophenyl)vinyl]phenyl}sulfonyl)pyridine

### Step 1

A mixture of 2-bromopyridine (100 mg, 0.633 mmol), sodium 4-bromophenylsulfinate (211 mg, 0.76 mmol) and copper(I) iodide (360 mg, 1.9 mmol) in dimethylsulfoxide (2 mL) was heated at 110°C for 3 hours. The cooled reaction mixture was partitioned between ethyl acetate and water. The organic layer was dried over MgSO₄ and evaporated *in vacuo.* The residue was purified by flash column chromatography on silica to give 2-[(4-bromophenyl)sulfonyl]pyridine.

### Step 2

50 mg (0.17 mmol) of this was combined with [(*E*)-2-(4-fluorophenyl)vinyl]boronic acid (27 mg, 0.16 mmol) and tetrakis(triphenylphosphine)palladium(0) (10 mg) in dioxan/2N cesium carbonate (2 mL/0.17 mL) in a 5mL microwave vial. The vial was heated to 150°C for 10 minutes in a microwave reactor. Saturated ammonium chloride was added and the products extracted into ethyl acetate (x2). The combined organic extracts were washed with brine, dried over MgSO₄ and concentrated *in vacuo.* The residue was purified by flash column chromatography on silica to give the title compound. δ_{H} (500 MHz, d⁶ DMSO): 8.69 (1H, d, J = 4.3 Hz), 8.20 (1H, d, J = 7.8 Hz), 8.13 (1 H, t, J = 7.7 Hz), 7.93 (2 H, d, J = 8.4 Hz), 7.81 (2 H, d, J = 8.4 Hz), 7.70-7.66 (3 H, m), 7.44 (1 H, d, J = 16.4 Hz), 7.29 (1 H, d, J = 16.5 Hz), 7.22 (2 H, t, J = 8.8 Hz).

### Example 2

### 4-{[6-({4-[(E)-2-(4-fluorophenyl)vinyl]phenyl}sulfonyl)pyridin-2-yl]methyl}morpholine

To a solution of 6-({4-[(*E*)-2-(4-fluorophenyl)vinyl]phenyl}sulfonyl)pyridine-2-carbaldehyde (prepared according to the method of Example 1 using 6-bromopyridine-2-carboxyldehyde in step 1,100 mg, 0.27 mmol) in methanol (0.8 mL) was added morpholine (0.05 mL, 0.54 mmol) and acetic acid (0.08 mL, 1.36 mmol) and the reaction was stirred at room temperature under nitrogen for 30 minutes. Sodium cyanoborohydride (17 mg, 0.27 mmol) was added and stirring continued at room temperature for 2 days. 1N sodium hydroxide and dichloromethane were added and the organic layer separated, washed with brine, dried over Na₂SO₄ and evaporated *in vacuo.* The residue was purified by flash column chromatography on silica, eluting with 1% methanol/dichloromethane, to yield the title compound (70 mg, 59%). δ_{H} (400 MHz, d⁶ DMSO): 8.11-8.05 (2 H, m), 7.92 (2 H, d, J = 8.4 Hz), 7.80 (2 H, d, J = 8.4 Hz), 7.70-7.63 (3 H, m), 7.44 (1 H, d, J = 16.5 Hz), 7.29 (1 H, d, J = 16.5 Hz), 7.23 (2 H, t, J = 8.8 Hz), 3.58 (2 H, s), 3.49 (4 H, t, J = 4.5 Hz), 2.30 (4 H, t, J = 4.3 Hz).

### Examples 3-5

The following 3 compounds were prepared according to the method of Example 1 using the appropriate halopyridine in step 1 and the appropriate styryl boronic acid in step 2.

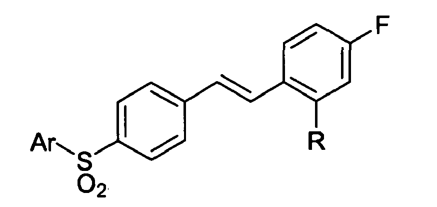

| Example | Ar | R | *m*/*z* (ES⁺) [MH⁺] |
|---|---|---|---|
| 3 | 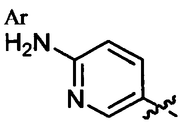 | H | 355 |
| 4 | 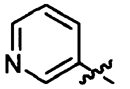 | H | 340 |
| 5 | 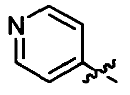 | F | 358 |

### Example 6

### 5-({4-[(E)-2-(2,4-difluorophenyl)vinyl]phenyl}sulfonyl)pyridin-2-amine

### Step 1

5-[(4-Bromophenyl)sulfonyl]pyridin-2-amine (prepared according to the method of Example 1 step 1, using 2-amino-5-bromopyridine, 948 mg, 3.03 mmol), tributyl(vinyl)tin (666 mg, 3.63 mmol) and tetrakis(triphenylphosphine)palladium(0) (100 mg) were combined in tetrahydrofuran (5 mL) and heated to 150°C for 10 minutes in a microwave reactor. The reaction mixture was dry-loaded onto silica and purified by flash column chromatography using ethyl acetate/isohexane to give 5-[(4-vinylphenyl)sulfonyl]pyridin-2-amine (0.82 g). δ_{H} (500 MHz, d⁶ DMSO): 8.40 (1 H, d, J = 2.4 Hz), 7.83 (2 H, d, J = 8.4 Hz), 7.74 (1 H, dd, J = 2.5, 8.9 Hz), 7.65 (2 H, d, J = 8.4 Hz), 7.05 (2 H, s), 6.78 (1 H, dd, J = 10.9, 17.6 Hz), 6.47 (1 H, d, J = 8.9 Hz), 5.97 (1 H, d, J = 17.6 Hz), 5.42 (1 H, d, J = 11.0 Hz).

### Step 2

5-[(4-Vinylphenyl)sulfonyl]pyridin-2-amine (Step 1,100 mg, 0.38 mmol), 2,4-difluoroiodobenzene (91 mg, 0.38 mmol), palladium(II) acetate (2 mg, 0.01 mmol) and tri-*o*-tolylphosphine (12 mg, 0.039 mmol) were taken up in acetonitrile/triethylamine (0.5 mL/0.5 mL) and the reaction heated to 170°C for 20 minutes in a microwave reactor. The reaction was diluted with ethyl acetate and washed with brine, dried over MgSO₄ and concentrated *in vacuo.* The residue was purified by MassLynx and preparative HPLC to give the title compound. δ_{H} (500 MHz, d⁶ DMSO): 7.66 (1H, d, J = 2.3 Hz), 7.17 (1H, dd, J = 2.4, 9.2 Hz), 7.11 (2 H, d, J = 8.5 Hz), 6.95-6.92 (4 H, m), 6.59 (1 H, d, J = 16.6 Hz), 6.44 (1 H, d, J = 16.6 Hz), 6.18-6.14 (3 H, m), 5.99 (1 H, d, J = 9.3 Hz).

### Example 7

### Methyl 2-({4-[(E)-2-(4-fluorophenyl)vinyl]phenyl}sulfonyl)nicotinate

### Step 1

To a suspension of sodium 4-bromophenylsulfinate (130 g, 0.53 mol) in water (600 mL) was added acrylonitrile (70 mL, 1.07 mol) and acetic acid (62 mL, 1.07 mol). The reaction was stirred for 1.5 hours at 100°C then cooled to room temperature. The solid was filtered off, washed thoroughly with water and dried over P₂O₅ to give 3-[(4-bromophenyl)sulfonyl]propanenitrile (125 g. 85%). δ_{H} (400 MHz, CDCl₃): 7.27-7.22 (4 H, m), 2.85 (2 H, t, J = 7.6 Hz), 2.30 (2 H, t, J = 7.6 Hz).

### Step 2

To a suspension of sodium acetate (54 g, 0.66 mol) and 4-fluorostyrene (90 g, 0.74 mol) in 1-methyl-2-pyrrolidinone (500 mL) was added 3-[(4-bromophenyl)sulfonyl]propanenitrile (Step 1, 90 g, 0.33 mol) and palladium(II) acetate (1.4 g, 6.2 mmol). The mixture was plunged into an oil-bath at 100°C and heated to 135°C for 20 minutes. The cooled reaction mixture was diluted with water and ethyl acetate and filtered through Hyflo®. The organic layer of the filtrate was washed with water (x3) then concentrated *in vacuo.* The residue was triturated with isohexane to give 3-({4-[(*E*)-2-(4-fluorophenyl)vinyl]phenyl}sulfonyl)propanenitrile (73 g, 71%). δ_{H} (360 MHz, CDCl₃): 7.88 (2 H, d, J = 8.0 Hz), 7.69 (2 H, d, J = 8.3 Hz), 7.51 (2 H, dd, J = 5.6, 8.3 Hz), 7.22 (1H, d, J = 15.0 Hz), 7.10-7.02 (3 H, m), 3.39 (2 H, t, J = 7.7 Hz), 2.83 (2 H, t, J = 7.7 Hz).

### Step 3

To a mixture of 3-({4-[(*E*)-2-(4-fluorophenyl)vinyl]phenyl}sulfonyl)propanenitrile (Step 2, 75 g, 0.24 mol) in tetrahydrofuran (1L) and methanol (500 mL) was added sodium methoxide (13 g, 0.24 mol). The mixture was stirred for 1 hour at room temperature then diluted with isohexane and diethyl ether. The solid was filtered off, triturated with isohexane and dried under vacuum to give sodium 4-[(*E*)-2-(4-fluorophenyl)vinyl]benzenesulfinate (66 g, 98%). δ_{H} (400 MHz, d⁶ DMSO): 7.65-7.61 (2 H, m), 7.51 (2 H, d, J = 8.1 Hz), 7.43 (2 H, d, J = 8.1 Hz), 7.25-7.15 (4 H, m).

### Step 4

Sodium 4-[(*E*)-2-(4-fluorophenyl)vinyl]benzenesulfinate (Step 3, 795 mg, 2.80 mmol), 2-bromonicotinic acid (563 mg, 2.80 mmol) and copper iodide (1.67 g, 8.40 mmol) were suspended in dimethylsulfoxide and heated to 130°C for 2 hours. The cooled reaction mixture was diluted with ethyl acetate and water and filtered through Hyflo®, washing the filter cake with more ethyl acetate. The organic layer was dried over MgSO₄ and evaporated. The residue was purified by flash column chromatography on silica, eluting with 1% acetic acid/ethyl acetate, to yield 2-({4-[(*E*)-2-(4-fluorophenyl)vinyl]phenyl}sulfonyl)nicotinic acid (760mg, 70%). δ_{H} (500 MHz, d⁶ DMSO): 8.70 (1 H, d, J = 4.2 Hz), 8.15 (1 H, d, J = 7.4 Hz), 7.94 (2 H, d, J = 8.3 Hz), 7.82 (2 H, d, J = 8.4 Hz), 7.73-7.66 (3 H, m), 7.45 (1 H, d, J = 16.4 Hz), 7.29 (1 H, d, J = 16.5 Hz), 7.22 (2 H, t, J = 8.8 Hz).

### Step 5

2-({4-[(*E*)-2-(4-Fluorophenyl)vinyl]phenyl}sulfonyl)nicotinic acid (Step 4,100 mg, 0.26 mmol) was dissolved in 1-methyl-2-pyrrolidinone (10 mL). 1,1'-Carbonyldiimidazole (47 mg, 0.29 mmol) was added and the reaction was stirred for one hour. The reaction was diluted with 2M ammonia in methanol and heated to 80°C for one hour. The cooled reaction mixture was poured into water and extracted into ethyl acetate. The organic extract was washed with brine (x3), dried over MgSO₄ and evaporated. The residue was purified by flash column chromatography on silica, eluting with 50% ethyl acetate/isohexane to yield the title compound (65mg, 62%). δ_{H} (500 MHz, d⁶ DMSO): 8.77 (1 H, dd, J = 1.4, 4.6 Hz), 8.21 (1 H, dd, J = 1.4, 7.8 Hz), 7.92 (2H, d, J = 8.5Hz), 7.83 (2H, d, J = 8.4 Hz), 7.77 (1 H, dd, J = 4.6, 7.8 Hz), 7.69 (2 H, dd, J = 5.6, 8.5 Hz), 7.45 (1 H, d, J = 16.4 Hz), 7.30 (1 H, d, J = 16.4 Hz), 7.23 (2 H, t, J = 8.8 Hz), 3.93 (3 H, s).

### Example 8

### [2-({4-[(E)-2-(4-fluorophenyl)vinyl]phenyl}sulfonyl)pyridin-3-yl]methanol

### Step 1

2-({4-[(*E*)-2-(4-Fluorophenyl)vinyl]phenyl}sulfonyl)nicotinic acid (Example 7 Step 4, 0.5g, 1.31 mmol) was suspended in thionyl chloride (2.3 g; 19.58 mmol) and heated to reflux for 1 hour. The solvent was removed *in vacuo* and the residue azeotroped with toluene to give 2-({4-(*E*)-2-(4-fluorophenyl)vinyl]phenyl}sulfonyl)nicotinoyl chloride (assume 1.31 mmol).

### Step 2

2-({4-(*E*)-2-(4-fluorophenyl)vinyl]phenyl}sulfonyl)nicotinoyl chloride (Step 1, 0.65 mmol) was dissolved in tetrahydrofuran (5 mL) and treated with sodium borohydride (992 mg, 2.6 mmol). The reaction was stirred for 1 hour before quenching with water and extracting into ethyl acetate. The residue after evaporation was purified by flash column chromatography on silica to give the title compound (146 mg, 60%) δ_{H} (500 MHz, d⁶ DMSO): 8.45 (1 H, d, J = 4.0 Hz), 8.25 (1 H, d, J = 7.9 Hz), 7.90 (2 H, d, J = 8.4 Hz), 7.82 (2 H, d, J = 8.3 Hz), 7.71-7.65 (3 H, m), 7.46 (1 H, d, J = 16.4 Hz), 7.31 (1 H, d, J = 16.4 Hz), 7.23 (2 H, t, J = 8.7 Hz), 5.63 (1H, t, J = 5.6 Hz), 5.03 (2 H, d, J = 5.5 Hz).

### Example 9

### 2-({4-[(E)-2-(4-fluorophenyl)vinyl]phenyl}sulfonyl)nicotinamide

2-({4-(*E*)-2-(4-Fluorophenyl)vinyl]phenyl}sulfonyl)nicotinoyl chloride (Example 8 Step 1, 0.65 mmol) in tetrahydrofuran (5 mL) was treated with concentrated ammonium hydroxide and stirred for one hour at room temperature. The reaction mixture was extracted with ethyl acetate. The organic layer was dried (MgSO₄) and evaporated. The residue was purified by flash column chromatography on silica to yield the title compound (0.055g, 24%). δ_{H} (500 MHz, d⁶ DMSO): 8.70-8.68 (1 H, m), 8.11 (1 H, s), 8.01-7.98 (3 H, m), 7.83 (2 H, d, J = 8.5 Hz), 7.80 (1 H, s), 7.72-7.70 (3 H, m), 7.48 (1 H, d, J = 16.5 Hz), 7.31 (1 H, d, J = 16.5 Hz), 7.26 (2 H, t, J = 8.8 Hz).

### Example 10

### 1-[2-{{4-[(E)-2-(4-fluorophenyl)vinyl]phenyl}sulfonyl)pyridin-3-yl]ethanol

### Step 1

[2-({4-[(*E*)-2-(4-Fluorophenyl)vinyl]phenyl}sulfonyl)pyridin-3-yl]methanol (Example 8, 0.68 g, 1.84 mmol) was suspended in dichloromethane and 4-methylmorpholine *N*-oxide (0.32 g, 2.76 mmol) was added to give a solution. Molecular sieves (4Å, 2 g, freshly activated by microwave) were added and the reaction stirred for 15 minutes. Tetrapropylammonium perruthenate (32 mg, 0.09 mmol) was added and the reaction stirred at room temperature for 30 minutes. The reaction mixture was concentrated while loading onto silica and purified by flash column chromatography, eluting with 25% ethyl acetate/isohexane, to yield 2-({4-[(*E*)-2-(4-fluorophenyl)vinyl]phenyl}sulfonyl)nicotinaldehyde (0.35 g, 52%). δ_{H} (500 MHz, CDCl₃): 11.16 (H, s), 8. 71 (1 H, dd, J= 4.7,1.7 Hz), 8.3 8 (1 H, dd, J = 1.7, 7.9 Hz), 8.03 (2 H, d, J = 8.5 Hz), 7.67 (2 H, d, J = 8.5 Hz), 7.59 ( 1 H, dd, J = 4.7, 7.8 Hz), 7.53-7.50 (2 H, m), 7.22 (1 H, d, J = 16.3 Hz), 7.10-7.03 (3 H, m).

### Step 2

2-({4-[(*E*)-2-(4-fluorophenyl)vinyl]phenyl}sulfonyl)nicotinaldehyde (Step 1, 350 mg, 0.95 mmol) in tetrahydrofuran (20 mL) was stirred with methyl magnesium bromide (3M in tetrahydrofuran; 0.954 ml, 2.86 mmol) for 10 minutes. The reaction was quenched with saturated ammonium chloride and the mixture was extracted with dichloromethane. The organic extract was dried over MgSO₄ and evaporated. The residue was purified by flash column chromatography on silica using 30% ethyl acetate/isohexane to yield the title compound (0.278g, 76%). δ_{H} (500 MHz, d⁶ DMSO): 8.40 (1H, d, J = 3.1 Hz), 8.29 (1 H, d, J = 7.9 Hz), 7.89 (2 H, d, J = 8.4 Hz), 7.82 (2 H, d, J = 8.4 Hz), 7.70 (2 H, dd, J = 5.7, 8.4 Hz), 7.65 (1 H, dd, J = 4.5, 7.9 Hz), 7.46 (1 H, d, J = 16.4 Hz), 7.32 (1 H, d, J =16.5 Hz), 7.23 (2 H, t, J = 8.8 Hz), 5.84-5.80 (1 H, m), 5.60 (1 H, d, J = 4.2 Hz), 1.42 (3 H, d, J = 6.3 Hz); *m*/*z* (ES⁺) 366 [(M-OH)⁺].

### Example 11

### [2-({4-[(E)-2-(2,4-difluorophenyl)vinyl]phenyl}sulfonyl)pyridin-3-yl]methanol

Prepared according to the procedures of Example 7 steps 1-4 (using 2,4-difluorostyrene in step 2), and Example 8. δ_{H} (500 MHz, d⁶ DMSO): 8.45 (1 H, d, J = 4.4 Hz), 8.25 (1 H, d, J = 7.9 Hz), 7.91-7.85 (5 H, m), 7.67 (1 H, dd, J = 4.6, 7.9 Hz), 7.41 (2 H, q, J = 13.5 Hz), 7.30 (1 H, t, J = 10.2 Hz), 7.16 (1H, t, J = 8.5 Hz), 5.63 ( H, t, J = 5.6 Hz), 5.03 (2 H, d, J = 5.6 Hz).

### Example 12

### 2-({4-[(E)-2-(2,4-difluorophenyl)vinyl]phenyl}sulfonyl)nicotinamide

Prepared from the aldehyde precursor fo Example 11 according to the method of Example 9. δ_{H} (500 MHz, d⁶ DMSO): 8.66 (1 H, dd, J = 1.4, 4.6 Hz), 8.07 (1 H, d, J = 13.7 Hz), 7.97 (3 H, t, J = 7.4 Hz), 7.89-7.83 (3 H, m), 7.76 (1 H, s), 7.72-7.66 (1 H, m), 7.39 (2 H, q, J=15.8 Hz), 7.30 (1 H, t, J = 10.2 Hz), 7.15 (1 H, t, J = 8.5 Hz).

### Example 13

### 1-[2-({4-[(E)-2-(4-fluorophenyl)vinyl]phenyl}sulfonyl)pyridin-3-yl]ethanone

Prepared by oxidation of Example 10 according to the method of Example 10 Step 1. δ_{H} (500 MHz, d⁶ DMSO): 8.71 (1 H, dd, J = 1.3, 4.5 Hz), 8.13 (1 H, dd, J = 1.3, 7.8 Hz), 7.90 (2 H, d, J = 8.4 Hz), 7.83 (2 H, d, J = 8.4 Hz), 7.75 (1H, dd, J = 4.6, 7.8 Hz), 7.69 (2 H, dd, J = 5.9, 8.4 Hz), 7.46 (1 H, d, J = 16.4 Hz), 7.30 (1 H, d, J = 16.4 Hz), 7.23 (2 H, t, J = 8.7 Hz), 2.67 (3 H, s).

### Example 14

### 2,2,2-trifluoro-1-[2-({4-[(E)-2-(4-fluorophenyl)vinyl]phenyl}sulfonyl)pyridin-3-yl]ethanol

2-({4-[(*E*)-2-(4-Fluorophenyl)vinyl]phenyl}sulfonyl)nicotinaldehyde (Example 10 Step 1, 100 mg, 0.27 mmol) was dissolved in trimethyl(trifluoromethyl)silane (0.5M in tetrahydrofuran, 1.09 ml, 0.55 mmol) and cesium fluoride (4 mg, 0.03 mmol) was added. The reaction was stirred for 16 hours at room temperature. The solvent was removed *in vacuo.* The residue was dissolved in dichloromethane and stirred with trifluoroacetic acid (1mL) for 16 hours. The reaction mixture was partitioned between ethyl acetate and saturated sodium bicarbonate solution. The organic extract was dried over MgSO₄ and evaporated. The residue was purified by flash colum chromatography on silica, eluting with 35% ethyl acetate/isohexane to give the title compound (38mg, 32%). δ_{H} (500 MHz, d⁶ DMSO): 8.57 (1 H, dd, J = 1.3,4.5 Hz), 8.28 (1 H, d, J = 8.1 Hz), 8.02 (2 H, d, J = 8.3 Hz), 7.65 (2 H, d, J = 8.4 Hz), 7.52-7.50 (3 H, m), 7.21 (1 H, d, J = 16.3 Hz), 7.10-7.02 (3 H, m), 6.75-6.69 (1 H, m), 3.39 (1 H, d, J = 5.4 Hz); *m*/*z* (ES⁺) 438 [MH⁺].

### Example 15

### 6-({4-[(E)-2-(4-fluoropbenyl)vinyl]phenyl}sulfonyl)-N-methylpyridine-2-carboxamide

Prepared from 6-({4-[(*E*)-2-(4-fluorophenyl)vinyl]phenyl}sulfonyl)pyridine-2-carbonyl chloride (prepared according to the method of Example 7 steps 1-4, using 4-bromopicolinic acid in step 4, and Example 8 Step 1) and methylamine according to the method of Example 9. δ_{H} (500 MHz, d⁶ DMSO): 8.56 (1 H, d, J = 4.7 Hz), 8.33-8.26 (2 H, m), 8.23 (1 H, d, J = 7.5 Hz), 8.15 (2 H, d, J = 8.4 Hz), 7.81 (2 H, d, J = 8.4 Hz), 7.68 (2 H, dd, J = 5.7, 8.4 Hz), 7.47 (1 H, d, J = 16.4 Hz), 7.29 (1 H, d, J = 16.4 Hz), 7.23 (2 H, t, J = 8.8 Hz), 2.85 (3 H, d, J = 4.7 Hz).

### Example 16

### Ethyl 2-({4-[(E)-2-(4-fluorophenyl)vinyl]phenyl}sulfonyl)nicotinate

2-({4-[(*E*)-2-(4-Fluorophenyl)vinyl]phenyl}sulfonyl)nicotinic acid (Example 7 Step 4, 0.5 g, 1.31 mmol) was suspended in dichloromethane (10mL) and oxalyl chloride (1.14 mL, 13.06 mmol) was added followed by one drop of *N,N*-dimethylformamide. The reaction was stirred until gas evolution ceased. Toluene was added and the reaction mixture was evaporated to dryness. Ethanol was added and the reaction was heated to reflux for 16 hours. The reaction was cooled and crystals of the title compound were filtered off and dried (0.4g, 74%). δ_{H} (500 MHz, d⁶ DMSO): 8.76 (1 H, d, J = 3.3 Hz), 8.20 (1 H, d, J = 6.6 Hz), 7.92 (2 H, d, J = 8.3 Hz), 7.83 (2 H, d, J = 8.4 Hz), 7.76 (1 H, dd, J = 4.6, 7.7 Hz), 7.69 (2 H, dd, J = 5.7, 8.4 Hz), 7.46 (1 H, d, J = 16.4 Hz), 7.30 (1 H, d, J = 16.4 Hz), 7.23 (2 H, t, J = 8.7 Hz), 4.40 (2 H, q, J = 7.1 Hz), 1.35 (3 H, t, J = 7.1 Hz).

### Example 17

### 2-[2-({4-[(E)-2-(4-fluorophenyl)vinyl]phenyl}sulfonyl)pyridin-3-yl]propan-2-ol

### Step 1

Lithium diisopropylamide (2M, 12.5 ml, 25 mmol) was dissolved in tetrahydrofuran (40 mL) and cooled to -78°C. 2-Bromopyridine (3.9 g, 25 mmol) was added dropwise and the reaction was stirred for 3 hours before adding acetone (1 mL, dried over freshly activated molecular sieves) and allowing to warm to room temperature. The reaction was quenched with saturated ammonium chloride and extracted with ethyl acetate. The organic layer was dried over MgSO₄ and evaporated. The residue was purified by flash column chromatography on silica, eluting with 20% ethyl acetate/isohexane, to give 2-(2-bromopyridin-3-yl)propan-2-ol (1.4 g, 26%). δ_{H} (500 MHz, d⁶ DMSO): 8.23 (1 H, dd, J = 1.9, 4.5 Hz), 8.19 (1 H, dd, J = 2.0, 7.8 Hz), 7.44 (1 H, dd, J = 4.5, 7.7 Hz), 5.43 (1 H, s), 2.12 (1 H, s), 1.64 (6 H, s).

### Step 2

2-(2-Bromopyridin-3-yl)propan-2-ol (Step 1) was reacted with sodium 4[(E)-2-(4-fluorophenyl)vinyl]benzenesulfinate according to the method of Example 7 Step 4. δ_{H} (500 MHz, d⁶ DMSO): 8.34 (1 H, dd, J = 1.4, 8.1 Hz), 8.29 (1 H, dd, J = 1.4, 4.4 Hz), 7.83 (2 H, d, J = 8.5 Hz), 7.78 (2 H, d, J = 8.5 Hz), 7.70 (2 H, dd, J = 5.6, 8.6 Hz), 7.56 (1 H, dd, J = 4.4, 8.1 Hz), 7.44 (1 H, d, J = 16.4 Hz), 7.31 (1 H, d, J = 16.5 Hz), 7.23 (2 H, t, J = 8.8 Hz), 5.51 (1 H, s), 1.77 (6 H, s).

### Example 18

### 2-[2-({4-[(E)-2-(2,4-difluorophenyl)vinyl]phenyl}sulfonyl)pyridin-3-yl]propan-2-ol

Prepared according to the method of Example 17 with sodium 4[(E)-2-(2,4-difluorophenyl)vinyl]benzenesulfinate in step 2. δ_{H} (500 MHz, d⁶ DMSO): 8.34 (1 H, dd, J = 1.3, 8.1 Hz), 8.29 (1 H, dd, J = 1.3, 4.4 Hz), 7.89 (1 H, q, J = 8.1 Hz), 7.85-7.81 (4 H, m), 7.56 (1 H, dd, J = 4.4, 8.1 Hz), 7.44-7.37 (2 H, m), 7.33-7.28 (1 H, m), 7.18-7.14 (1 H, m), 5.51 (1 H, s), 1.77 (6 H, s).

### Example 19

### 1-[2-({4-[(E)-2-(2,4-difluorophenyl)vinyl]phenyl}sulfonyl)pyridin-3-yl]ethanol

Sodium 4-[(*E*)-2-(2,4-difluorophenyl)vinyl]benzenesulfinate (prepared according to the method of Example 7 Steps 1-3 using 2,4-difluorostyrene in step 2, 302 mg, 1 mmol) and 1-(2-chloropyridin-3-yl)ethanone (prepared according to the method in patent WO 2003094918, 155 mg, 1 mmol) were dissolved in dimethylsulfoxide (4 mL) and heated to 160°C in a microwave for 1 hour. The cooled reaction was poured into water. Extraction with ethyl acetate was attempted but the product did not dissolve so the solid residue and ethyl acetate were washed in a flask and azeotroped with toluene to give 399 mg. This was suspended in tetrahydrofuran (5 mL) and ethanol (5 mL). Excess sodium borohydride was added and the reaction was stirred for two hours before being poured into water and extracted with ethyl acetate. The organic layer was dried over MgSO₄ and evaporated *in vacuo.* The residue was purified by flash column chromatography on silica, eluting with 30% ethyl acetate/isohexane to give the title compound (58 mg, 14%). δ_{H} (500 MHz, d⁶ DMSO): 8.40 (1 H, d, J = 3.2 Hz), 8.29 (1 H, d, J = 7.8 Hz), 7.90-7.85 (5 H, m), 7.65 (1 H, dd, J = 4.4, 7.8 Hz), 7.46-7.38 (2 H, m), 7.31 (1 H, t, J = 9.8 Hz), 7.16 (1 H, t, J = 7.8 Hz), 5.81 (1 H, t, J = 4.6 Hz), 5.60 (1 H, d, J = 4.0 Hz), 1.41 (3 H, d, J = 6.2 Hz).

### Example 20

### 1-[2-({4-[(E)-2-(2,4-difluorophenyl)vinyl]phenyl}sulfonyl)pyridin-3-yl]propan-1-ol

Prepared from propionaldehyde and 2-bromopyridine according to the method of Example 17. *m*/*z* (ES⁺) 398 [(M-OH)⁺].

### Example 21

### 1-[2-({4-[(E)-2-(4-fluorophenyl)vinyl]phenyl}sulfonyl)pyridin-3-yl]propan-1-ol

Prepared from propionaldehyde and 2-bromopyridine according to the method of Example 17. *mlz* (ES⁺) 380 [(M-OH)⁺].

### Example 22

### 1-[2-({4-[(E)-2-(4-fluorophenyl)vinyl]phenyl}sulfonyl)pyridin-3-yl]cyclobutanol

Prepared from cyclobutanone and 2-bromopyridine according to the method of Example 17. *mlz* (ES⁺) 392 [(M-OH)⁺].

### Example 23

### 1-[2-({4-[(E)-2-(2,4-difluorophenyl)vinyl]phenyl}sulfonyl)pyridin-3-yl]cyclobutanol

Prepared from cyclobutanone and 2-bromopyridine according to the method of Example 18. *m*/*z* (ES⁺) 410 [(M-OH)⁺]

### Example 24

### 3-({4-[(E)-2-4-fluorophenyl)vinyl]phenyl}sulfonyl)-N,N-dimethylpyridin-2-amine

Prepared according to the method of Example 7 Steps 1-4 using 2-(dimethylamino)-3-iodopyridine (prepared according to Tet. Lett., 1997, 38(48), 8331) at 110°C in Step 4. *mlz* (ES⁺) 383 [MH⁺]

### Example 25

### 2-fluoro-3-({4-[(E)-2-(4-fluorophenyl)vinyl]phenyl}sulfonyl)pyridine

A solution of lithium diisopropylamide (2M, 8 mL, 16 mmol) in tetrahydrofuran (40 mL) was cooled to - 78°C and 2-fluoropyridine (1.4 mL, 16 mmol) added. The reaction was stirred at -78°C for 4 hours. Iodine (4.1 g, 16 mmol) in tetrahydrofuran (12 mL) was added and the reaction stirred for 1 hour then quenched with water/tetrahydrofuran (1:1, 2 mL) at -78°C. The mixture was warmed to 0°C and partitioned between ethyl acetate and water. The organic layer was washed with brine, dried over MgSO₄ and evaporated to give 2-fluoro-3-iodopyridine. This was treated according to the method of Example 7 Step 4 at 110°C to give the title compound. *m*/*z* (ES⁺) 358 [MH⁺].

### Example 26

### 3-{4-[(E)-2-(4-fluorophenyl)vinyl]phenyl}sulfonyl)-N-methylpyridin-2-amine

2-Fluoro-3-({4-[(*E*)-2-(4-fluorophenyl)vinyl]phenyl)sulfonyl)pyridine (Example 25, 50 mg, 0.14 mmol) was dissolved in methylamine (1M in tetrahydrofuran) and heated to 150°C for 10 minutes in a microwave reactor. The solvent was removed *in vacuo* and the residue recrystallised from ethyl acetate/isohexane to give the title compound as a white solid. δ_{H} (500 MHz, d⁶ DMSO): 8.28 (1 H, dd, J = 1.6, 4.7 Hz), 8.08 (1 H, dd, J = 1.6, 7.7 Hz), 7.99 (2 H, d, J = 8.4 Hz), 7.78 (2 H, d, J = 8.4 Hz), 7.67 (2 H, dd, J = 5.7, 8.6 Hz), 7.44 (1 H, d, J = 16.4 Hz), 7.27 (1 H, d, J = 16.5 Hz), 7.22 (2 H, t, J = 8.8 Hz), 6.89 (1 H, q, J = 4.5 Hz), 6.73 (1 H, dd, J = 4.7, 7.8 Hz), 2.88 (3 H, d, J = 4.6 Hz); *mlz* (ES⁺) 369 [MH⁺].

### Example 27

### Methyl 3-({4-[(E)-2-(4-fluorophenyl)vinyl]phenyl}sulfonyl)pyridine-2-carbozylate

### Step 1

3-Bromopicolinic acid (1.93 g, 8.65 mmol) was heated to reflux in thionyl chloride (30.9 g, 259 mmol) for one hour. The solvent was removed *in vacuo* and the residue azeotroped with toluene. The residue was dissolved in methanol and heated to reflux for 3 hours. The solvent was removed *in vacuo* and the residue taken up into ethyl acetate. The organic extract was washed with saturated sodium bicarbonate solution, dried over MgSO₄ and evaporated. The residue was purified by flash column chromatography on silica, eluting with 35% ethyl acetate/isohexane, to yield methyl 3-bromopyridine-2-carboxylate as an oil (1.33g, 72%). δ_{H} (500 MHz, d⁶ DMSO): 8.60 (1 H, dd, J = 1.2, 4.6 Hz), 8.23 (1 H, dd, J = 1.1, 8.2 Hz), 7.51 (1 H, dd, J = 4.6, 8.2 Hz), 3.89 (3 H, s).

### Step 2

The title compound was prepared from methyl 3-bromopyridine-2-carboxylate (Step 1) according to the method of Example 7 Step 4 at 110°C. *mlz* (ES⁺) 398 [MH⁺].

### Example 28

### 3-({4-[(E)-2-(4-fluorophenyl)vinyl]phenyl}sulfonyl)pyridine-2-carboxamide

### Step 1

Methyl 3-({4-[(*E*)-2-(4-fluorophenyl)vinyl]phenyl}sulfonyl)pyridine-2-carboxylate (Example 27, 1.15 g, 2.9 mmol) was dissolved in tetrahydrofuran (50 mL) and lithium hydroxide (0.7 g, 28.97 mmol) was added, followed by water (10 mL). The reaction was stirred for 16 hours at room temperature. Sodium hydroxide (2M, 25 mL) was added followed by water (500 mL). The mixture was extracted with ethyl acetate. The aqueous layer was acidified to pH 3-4 with hydrochloric acid then extracted twice with ethyl acetate. The combined organic layers were dried over MgSO₄ and evaporated. The residue was triturated with diethyl ether/isohexane to yield 3-({4-[(*E*)-2-(4-fluorophenyl)vinyl]phenyl}sulfonyl)pyridine-2-carboxylic acid (1.07 g, 96%). δ_{H} (500 MHz, d⁶ DMSO): 8.82 (1 H, dd, J = 1.2, 4.7 Hz), 8.55 (1 H, dd, J = 1.3,8.2 Hz), 7.97 (2 H, t, J = 7.1 Hz), 7.81 (2 H, d, J = 8.4 Hz), 7.75 (1 H, dd, J = 4.8,8.2 Hz), 7.67 (2 H, dd, J = 5.7, 8.4 Hz), 7.46 (1H, d, J = 16.4 Hz), 7.27 ( H, d, J = 16.4 Hz), 7.22 (2 H, t, J = 8.7 Hz), 3.42 ( H, s).

### Step 2

The title compound was prepared from 3-((4-[(*E*)-2-(4-fluorophenyl)vinyl]phenyl) sulfonyl)pyridine-2-carboxylic acid (Step 1) according to the method of Example 9. *mlz* (ES⁺) 383 [MH⁺].

### Example 29

### 2-({4-[(E)-2-(4-fluorophenyl)vinyl]phenyl}sulfonyl)pyridin-3-amine

Iron powder (0.83 g, 14.8 mmol) was added portionwise to a stirred solution of 2-({4-[(*E*)-2-(4-fluorophenyl)vinyl]phenyl}sulfonyl)-3-nitropyridine (prepared according to the method of Example 7 Step 4 using 2-bromo-3-nitropyridine at 90°C, 1.14 g, 2.96 mmol) in acetic acid (9 mL). The reaction was heated at 70°C for 3 hours then cooled and concentrated by a stream of nitrogen overnight. The residue was partitioned between water and ethyl acetate and the mixture filtered through Hyflo®. The organic layer was washed with brine, dried over MgSO₄ and concentrated *in vacuo* to give the title compound (582 mg, 55%). δ_{H} (500 MHz, d⁶ DMSO): 7.90 (2 H, d, J = 8.3 Hz), 7.79-7.77 (3 H, m), 7.68 (2 H, dd, J = 5.6, 8.5 Hz), 7.42 (1 H, d, J = 16.4 Hz), 7.29-7.21 (5 H, m), 6.42 (2 H, s).

### Examples 30, 31

The following 2 compounds were prepared from 2-amino-3-bromopyridine according to the method of Example 7 steps 1-4 (step 4 at 110°C), using the appropriate fluorinated styrene in step 2.

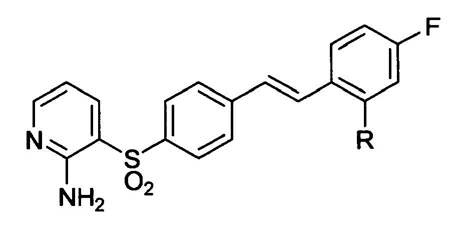

| Example | R | *m*/*z* (ES⁺) [MH⁺] |
|---|---|---|
| 30 | H | 355 |
| 31 | F | 373 |

### Example 32

### 1-[2-({4-[(E)-2-(2,4-difluorophenyl)vinyl]phenyl}sulfonyl)pyridin-3-yl]ethanone

### Step 1

2-chloronicotinic acid (1.57 g, 10 mmol) was suspended in dichloromethane. Methyl magnesium bromide (3M in diethyl ether, 10 mL, 30 mmol) was added. The reaction was stirred for 16 hours at room temperature then quenched with saturated ammonium chloride and extracted into ethyl acetate. The organic layer was washed with sodium hydrogencarbonate, dried over MgSO₄ and evaporated. The residue was purified by flash column chromatography on silica, eluting with ethyl acetate/isohexane, to yield 1-(2-chloropyridin-3-yl)ethanone (0.82g, 53%). δ_{H} (500 MHz, CDCl₃): 8.47 (1 H, dd, J = 1.9, 4.7 Hz), 7.88 (1 H, dd, J = 1.9, 7.6 Hz), 7.32 (1 H, dd, J = 4.8, 7.6 Hz), 2.67 (3 H, s).

### Step 2

Sodium 4-[(*E*)-2-(2,4-difluorophenyl)vinyl]benzenesulfinate (prepared according to the method of Example 7 Steps 1-3 using 2,4-difluorostyrene in step 2, 3 g, 9.93 mmol) and 1-(2-chloropyridin-3-yl)ethanone (Step 1,1.54 g, 9.93 mmol) were dissolved in dimethylsulfoxide (10 mL) and heated to 160°C for 2 hours. The reaction was poured into water and extracted with ethyl acetate, dichloromethane and methanol mixtures. The organic layers were dried over MgSO₄ and evaporated. The residue was purified by flash column chromatography on silica, eluting with ethyl acetate/isohexane and 5% diethyl ether/dichloromethane. The solid obtained was recrystallised from ethyl acetate to yield the title compound (1.3g, 33%). δ_{H} (500 MHz, d⁶ DMSO): 8.74-8.71 (1 H, m), 8.15-8.12 (1 H, m), 7.91-7.84 (5 H, m), 7.79-7.73 (1 H, m), 7.46-7.34 (2 H, m), 7.31-7.26 (1 H, m), 7.16-7.11 (1 H, m), 2.67 (3 H, s).

### Example 33

### 2-[2-({4-[(E)-2-(2-fluorophenyl)vinyl]phenyl}sulfonyl)pyridin-3-yl]propan-2-ol

The title compound was prepared according to the method of Example 7 using 2-fluorostyrene in Step 2 and 2-(2-bromopyridin-3-yl)propan-2-ol (Example 17 Step 1) in Step 4. δ_{H}, (500 MHz, d⁶ DMSO): 8.34 (1 H, dd, J = 1.3, 8.0 Hz), 8.29 (1 H, dd, J = 1.5,4.3 Hz), 7.84-7.81 (5 H, m), 7.57-7.55 (1H, m), 7.49-7.41 (2 H, m), 7.39-7.34 (1 H, m), 7.26-7.23 (2 H, m), 5.51 (1 H, s), 1.77 (6 H, s).

### Examples 34, 35

### (1R)- and (1S)-1-[2-({4-[(E)-2-(2,4-difluorophenyl)vinyl]phenyl}sulfonyl)pyridin-3-yl]ethanol

1-[2-({4-[(*E*)-2-(2,4-Difluorophenyl)vinyl]phenyl}sulfonyl)pyridin-3-yl]ethanone (Example 32, 0.3g, 0.75 mmol) was dissolved in a mixture of dichloromethane and methanol and cooled to 0°C. Sodium borohydride was added and the reaction allowed to warm to room temperature. The reaction mixture was partitioned between ethyl acetate and water. The organic layer was dried over MgSO₄ and evaporated. The residue was recrystallised from ethyl acetate/isohexane to yield 1-[2-({4-[(*E*)-2-(2,4-difluorophenyl)vinyl]phenyl}sulfonyl)pyridin-3-yl]ethanol (racemic, 0.19g, 63%). 90 mg of this was separated into its enantiomers by chiral SFC: Chiralcel OJ-H column (250 x 10mm i.d.), mobile phase CO₂/MeOH 35/65, flow rate 10ml/min.
Isomer 1: δ_{H} (500 MHz, d⁶ DMSO): 8.41-8.39 (1 H, m), 8.29 (1 H, dd, J = 1.9, 8.5 Hz), 7.91-7.85 (5 H, m), 7.66-7.64 (1H, m), 7.46-7.38 (2 H, m), 7.31 (1 H, t, J = 10.1 Hz), 7.16 (1 H, t, J = 8.2 Hz), 5.82-5.80 (1 H, m), 5.60-5.59 (1 H, m), 1.41 (3 H, dd, J = 1.6, 6.0 Hz).
Isomer 2: δ_{H} (500 MHz, d⁶ DMSO): 8.40 (1H, d, J = 4.2 Hz), 8.28 (1 H, d, J = 7.4 Hz), 7.91-7.85 (5 H, m), 7.67-7.64 (1H, m), 7.46-7.38 (2H, m), 7.31 (1H, t, J = 10.1 Hz), 7.17 (1H, t, J = 8.4 Hz), 5.83-5.79 (1H, m), 5.60-5.59 (1 H, m), 1.41 (3H, d, J = 6.1Hz).

### Example 36

### 1-[3-({4-[(E)-2-(4-fluorophenyl)vinyl]phenyl}sulfonyl)pyridin-4-yl]ethanone

### Step 1

3-Bromoisonicotinic acid (3.02 g, 15 mmol) was dissolved in dichloromethane (20 mL). Oxalyl chloride (9.53 g, 75 mmol) was added. After gas evolution had ceased (∼1 hour), the solvent was removed *in vacuo* and the residue redissolved in dichloromethane. *N*,*O*-dimethylhydroxylamine hydrochloride (2.94 g, 30 mmol) was added followed by triethylamine (4.55 g, 45 mmol) and the reaction was stirred for one hour at room temperature. The reaction mixture was diluted with ethyl acetate. The organic layer was washed with brine, dried over MgSO₄ and evaporated to yield 3-bromo-*N-*methoxy-*N*-methylisonicotinamide, 1.2 g of which was dissolved in THF (10 mL) and cooled to 0°C. Methyl magnesium bromide (4.92 ml, 9.84 mmol) was added and the reaction was allowed to warm to room temperature and stirred for two hours before being quenched with saturated aqueous ammonium chloride solution and extracted with ethyl acetate. The organic layer was dried over MgSO₄ and evaporated. The residue was purified by flash column chromatography on silica to yield 1-(3-bromopyridin-4-yl)ethanone (0.8g, 82%). δ_{H} (500 MHz, CDCl₃): 8.78 (1 H, s), 8.60 (1 H, d, J = 4.5 Hz), 7.28 (1 H, d, J = 3.8 Hz), 2.61 (3 H, d, J = 1.7 Hz).

### Step 2

The title compound was prepared from 1-(3-bromopyridin-4-yl)ethanone (Step 1) according to the method of Example 7 Step 4. δ_{H} (500 MHz, d⁶ DMSO): 9.27 (1 H, s), 8.95 (1H, d, J = 4.9 Hz), 7.92 (2 H, d, J = 8.4 Hz), 7.82 (2 H, d, J = 8.4 Hz), 7.70-7.67 (3 H, m), 7.47 (1 H, d, J = 16.4 Hz), 7.29 (1 H, d, J = 16.5 Hz), 7.23 (2 H, t, J = 8.7 Hz), 2.62 (3 H, s).

### Example 37

### (1R,S)-1-[3-({4-[(E)-2-(4-fluorophenyl)vinyl]phenyl}sulfonyl)pyridin-4-yl]ethanol

Prepared from 1-[3-({4-[(*E*)-2-(4-fluorophenyl)vinyl]phenyl)sulfonyl)pyridin-4-yl]ethanone (Example 36) according to the method of Example 34. δ_{H} (500 MHz, d⁶ DMSO): 9.11 (1 H, s), 8.84 (1 H, d, J = 5.1 Hz), 7.92 (2 H, d, J = 8.4 Hz), 7.82 (2H, d, J = 8.4 Hz), 7.76 (1 H, d, J = 5.1 Hz), 7.69 (2 H, dd, J = 5.7, 8.4 Hz), 7.46 (1 H, d, J = 16.4 Hz), 7.30 (1 H, d, J = 16.5 Hz), 7.23 (2 H, t, J = 8.8 Hz), 5.57 (1 H, d, J = 2.3 Hz), 5.36 (1 H, q, J = 6.6 Hz), 1.12 (3 H, d, J = 6.2 Hz).

### Example 38

### N-[2-({4-[(E)-2-(4-fluorophenyl)vinyl)phenyl}sulfonyl)pyridin-3-yl]methane sulfonamide

Sodium hydride (60% dispersion in mineral oil, 23 mg, 0.56 mmol) was added to a solution of 2-({4-[(*E*)-2-(4-fluorophenyl)vinyl]phenyl}sulfonyl)pyridin-3-amine (Example 29, 100 mg, 0.28 mmol) in *N,N-*dimethylformamide (1.4 mL) and stirred at room temperature for 10 minutes. Methanesulfonyl chloride (44 µL, 0.56 mmol) was added and the reaction stirred for 1 hour. A further 110 µL of methanesulfonyl chloride and 57 mg of sodium hydride were added and the reaction stirred for 24 hours. The mixture was purified by flash column chromatography on silica, eluting with ethyl acetate then 10% ethanol/ethyl acetate then 10% methanol/dichloromethane, followed by trituration with hot isohexane and washing with 50% diethyl ether/isohexane to give the title compound as an off-white solid. δ_{H} (400 MHz, d⁶ DMSO): 8.17 (1 H, d, J = 2.6 Hz), 7.82 (2 H, d, J = 8.3 Hz), 7.75-7.67 (5 H, m), 7.51-7.45 (2 H, m), 7.41 (1 H, d, J = 16.4 Hz), 7.30-7.20 (3 H, m), 2.92 (3 H, s).

### Example 39

### 2-({4-[(E)-2-(4-fluorophenyl)vinyl]phenyl}sulfonyl)nicotinonitrile

2-Chloronicotinonitrile (570 mg, 4.2 mmol) and sodium 4-[(*E*)-2-(4-fluorophenyl)vinyl]benzenesulfinate (Example 7 Step 3, 1.5 g, 5.4 mmol) were combined in dimethylsulfoxide (8.4 mL) and heated to 80°C under nitrogen for 12 hours. The cooled reaction mixture was partitioned between ethyl acetate and water. The organic layer was washed with brine, dried over Na₂SO₄ and evaporated *in vacuo.* The residue was dissolved in (75 mL) and water (ca. 30 mL) added dropwise until a precipitate formed. The solid was removed by filtration and dried under vacuum to give the title compound (720 mg, 47%). δ_{H} (500 MHz, d⁶ DMSO): 8.89 (1 H, t, J = 2.3 Hz), 8.64 (1 H, dd, J = 1.3, 7.9 Hz), 7.98 (2 H, d, J = 8.4 Hz), 7.87 (3 H, t, J = 7.6 Hz), 7.70 (2 H, dd, J = 5.7, 8.5 Hz), 7.49 (1 H, d, J = 16.5 Hz), 7.32 (1 H, d, J = 16.4 He), 7.23 (2 H, t, J = 8.8 Hz).

### Example 40

### N-{[2-({4-[(E)-2-(4-fluorophenyl)vinyl]phenyl}sulfonyl)pyridin-3-yl]methyl}propane-2-sulfinamide

### Step 1

To a cooled (-40°C) suspension of 2-chloro-3-cyanopyridine (5 g, 36 mmol) in toluene (18 mL) was added diisobutylaluminium hydride (1M in toluene, 36 mL, 36 mmol). The reaction was stirred at -40°C for 30 minutes then allowed to warm to room temperature. The solution was poured into a mixture of conc. H₂SO₄ )12 (mL) and ice-cold water (100 mL) and stirred vigorously for 2 hours. The mixture was extracted with toluene. The combined organic layers were washed with water, saturated aqueous sodium hydrogencarbonate and water, dried over Na₂SO₄ and evaporated *in vacuo* to give 2-chloronicotinaldehyde (4.06 g, 80%). δ_{H} (400 MHz, d⁶ DMSO): 10.27 (1 H, s), 8.66 (1 H, dd, J = 2.0, 4.7 Hz), 8.24 (1 H, dd, J = 2.1, 7.7 Hz), 7.63 (1 H, dd, J = 4.6, 7.3 Hz).

### Step 2

2-({4-[(*E*)-2-(4-Fluorophenyl)vinyl]phenyl}sulfonyl)nicotinaldehyde was prepared from 2-chloronicotinaldehyde (Step 1) and sodium 4-[(*E*)-2-(4-fluorophenyl)vinyl]benzenesulfinate (Example 7 Step 3) according to the method of Example 39.

### Step 3

To a solution of 2-({4-[(*E*)-2-(4-fluorophenyl)vinyl]phenyl}sulfonyl)nicotinaldehyde (Step 2, 250 mg, 0.68 mmol) in tetrahydrofuran (1.5 mL) was added 2-methyl-2-propanesulfinamide (90 mg, 0.75 mmol) and titanium(IV) ethoxide (0.28 mL, 1.36 mmol). The reaction was heated to reflux for 7 hours. The cooled reaction mixture was poured into brine and ethyl acetate added. The mixture was stirred for 10 minutes then the organic layer was dried over Na₂SO₄ and evaporated *in vacuo.* The residue was taken in dichloromethane (5 mL) and methanol (2 mL), and sodium borohydride (179 mg, 4 mmol) added portionwise. The reaction mixture was stirred for 10 minutes then partitioned between dichloromethane and water. The organic layer was dried over Na₂SO₄ and concentrated *in vacuo.* The residue was purified by flash column chromatography on silica, eluting with 90% ethyl acetate/isohexane, to give the title compound (198 mg, 62%). δ_{H} (400 MHz, d⁶ DMSO): 8.46 (1 H, dd, J = 1.6,4.8 Hz), 8.21 (1 H, dd, J = 1.4, 8.0 Hz), 7.92 (2 H, d, J = 8.5 Hz), 7.84 (2 H, d, J = 8.5 Hz), 7.72-7.66 (3 H, m), 7.48 (1 H, d, J = 16.5 Hz), 7.32 (1 H, d, J = 16.5 Hz), 7.24 (2 H, t, J = 8.9 Hz), 5.95 (1 H, t, J = 6.5 Hz), 4.82-4.70 (2 H, m), 1.17 (9 H, s).

### Example 41

### {[2-({4-[(E)-2-(4-fluorophenyl)vinyl]phenyl}sulfonyl)pyridin-3-yl]methyl}amine

*N*-([2-({4-[(*E*)-2-(4-Fluorophenyl)vinyl]phenyl}sulfonyl)pyridin-3-yl]methyl}propane-2-sulfinamide (Example 40, 190 mg, 0.4 mmol) was dissolved in methanol (3 mL) and HCl (4N in dioxan) was added. The reaction was stirred at room temperature overnight under nitrogen. The solvent was removed *in vacuo.* The residue was washed with diethyl ether and dried to give the title compound as the hydrochloride salt (150 mg, 92%). δ_{H} (400 MHz, d⁶ DMSO): 7.77 (1 H, dd, J = 1.4, 4.6 Hz), 7.29 (1 H, d, J = 7.8 Hz), 7.19 (2 H, d, J = 8.5 Hz), 6.99 (2 H, d, J = 8.5 Hz), 6.87-6.81 (3 H, m), 6.59 (1 H, d, J = 16.4 Hz), 6.40 (1 H, d, J = 16.4 Hz), 6.29 (2 H, t, J = 8.8 Hz), 3.83 (2 H, s).

### Example 42

### 2-({4-[(E)-2-(4-fluorophenyl)vinyl]phenyl}sulfonyl)-3-(methylsulfonyl)pyridine

A solution of 3-amino-2-chloropyridine (12.2 g, 0.1 mol) in tetrafluoroboric acid (50%, 40 mL) and ethanol (95%, 75 mL) was cooled to 5°C. Sodium nitrite (6.9 g, 0.1 mol) in water (20 ml) was added in one portion. Diethyl ether (100 mL) was added and the resulting precipitate removed by filtration then dissolved in acetonitrile (200 mL) and cooled to 0°C. Sodium thiomethoxide was added portionwise and the reaction stirred at room temperature for 2 hours. The reaction mixture was filtered through Hyflo® and the filtrate concentrated then passed through a plug of silica, eluting with 20% ethyl acetate/isohexane, to give 2-chloro-3-(methylthio)pyridine (1.5 g, 9.37 mmol). This was dissolved in dichloromethane (50 mL) and 3-chloroperoxybenzoic acid (77%, 5.2 g, 0.02 mol) was added. The reaction was stirred for 5 hours at room temperature. Calcium hydroxide (1 g, 0.014 mol) was added and the suspension stirred for 15 minutes then filtered through Hyflo®, washing with dichloromethane. The filtrate was concentrated *in vacuo.* The residue was triturated with diethyl ether/isohexane to give the title compound as a yellow solid (0.9 g).

### Example 43

### [2-({4-[(E)-2-(4-fluorophenyl)vinyl]phenyl}sulfonyl)-3-thienyl]methanol

Prepared according to the method of Example 7, Step 4 using (2-bromo-3-thienyl)methanol (itself prepared according to WO2004/065384A1). ¹H NMR (400 MHz, DMSO): δ 8.01 (1H, d, *J* 5.1), 7.91 (2H, d, *J* 8.5), 7.82 (2H, d, *J* 8.5), 7.70 (2H, dd, *J* 5.6, 8.7), 7.45 (1H, d, *J* 16.5), 7.32-7.22 (4H, m), 5.46 (1H, t, *J* 5.9), 4.67 (2H, d, *J* 5.9). *mlz* (ES⁺) 357 [(M-OH)⁺].

## Claims

1. A compound of formula I: 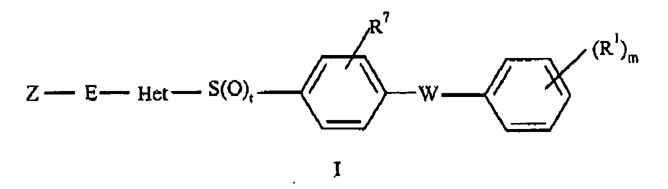 or a pharmaceutically acceptable salt thereof; wherein:
m is 0, 1, 2 or 3;
t is 1 or 2;
Het represents a pyridine or thiophene ring bearing 0, 1 or 2 R² substituents;
W represents -CR³R⁴-CR⁵R⁶, -CR³=CR⁵- or -C≡C- where R³, R⁴, R⁵, and R⁶ are selected from H, OH and F but not more than one of R³, R⁴, R⁵, and R⁶ is other than H; or R³ and R⁴ together or R⁵ and R⁶ together complete a keto group; or R⁴ and R⁶ together complete a cyclopropyl ring;
E represents a chemical bond or a straight or branched alkylene chain containing from 1 to 4 carbon atoms, optionally incorporating an oxygen atom to form an ether linkage;
Z is selected from halogen, CN, nitro, CF₃, OCF₃, -R^{a}, -OR^{a}, -SR^{a}, -SOR^{a}, -SO₂R^{a}; -SO₂NR^{a}R^{b}, -NR^{a}R^{b}, -NR^{a}COR^{b}, -NR^{a}CO₂R^{b}, -NR^{a}CO₂NR^{a}R^{b}, -NR^{a}S(O)₁R^{a}, -NR^{a}SO₂NR^{a}R^{b}, -COR^{a}, -CO₂R^{a}, -CONR^{a}R^{b}, -CH=NOR^{a} or a five- or six-membered heteroaromatic ring optionally bearing up to 2 substituent selected from halogen, CN, CF₃, C₁₋₆alkyl, C₁₋₆alkoxy, C₁₋₆alkylthio, amino, C₁₋₆alkylamino and di(C₁₋₆)alkylarnino;
R^{a} and R^{b} independently represent H or a hydrocarbon group of up to 7 carbon atoms which is optionally substituted with up to 3 fluorine atoms and optionally with Cl, Br, CN, OH, C₁₋₄alkoxy, C₁₋₄ alkylthio, amino, C₁₋₄alkylamino or di(C₁₋₄)alkylamino; or R^{a} and R^{b}, when linked through a nitrogen atom, together represent the residue of a heterocyclic ring of 4, 5 or 6 members, optionally bearing up to 3 substituents selected from halogen, CN, CF₃, oxo, OH, C₁₋₄alkyl and C₁₋₄alkaxy;
each R¹ independently represents halogen, CN, CF₃, OCF₃, C₁₋₆ alkyl, OH, benzylthio, C₁₋₆ alkoxy or hydroxymethyl;
each R² independently represents halogen, CN, CONH₂, C₁₋₄alkyl or C₁₋₄alkoxy;
and R⁷ represents H, halogen, CN, CF₃, OR^{a}, CO₂R^{a}, CONR^{a}R^{b}, NR^{a}R^{b} or C₁₋₄alkyl which is optionally substituted with halogen, CN, CF₃, OR^{a}, CO₂R^{a}, CONR^{a}R^{b} or NR^{a}R^{b}.

2. A compound according to claim 1 of Formula II or Formula III: 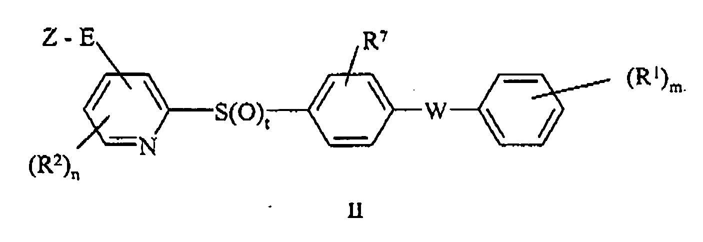 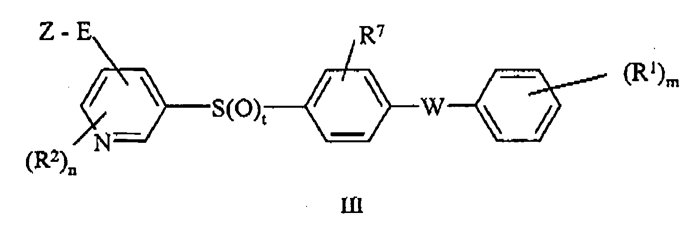 or a pharmaceutically acceptable salt thereof;
wherein n is 0, 1 or 2 and the other variables are as defined in claim 1.

3. A compound according to claim 2 wherein the moiety, Z-E- is attached at a ring position which is adjacent to the point of attachment of the -S(O)ₜ- moiety.

4. A compound according to claim 2 wherein the moiety Z-E- is attached at a ring position adjacent to the ring nitrogen.

5. A compound according to any previous claim wherein Z-E- is selected from H, isopropyl, 2-cyanoethyl, hydroxymethyl, 1-hydroxyethyl, 2-hydroxyethyl, 1-hydroxypropyl, 1-hydroxy-l-methylethyl, 1-hydroxy-2,2,2-trifluoroethyl, 1-hydroxycyclobutyl, CO₂Me, CO₂Et, CONH₂, CONHMe, COCH₃, NH₂, NHMe, NMe₂, NHSO₂Me, SO₂Me, CN, CH₂NH₂, CH₂NHSO^{t}Bu, CH₂NHCOMe, morpholin-4-yl and morpholin-4-ylmethyl.

6. A compound according to any previous claim wherein (R¹)ₘ represents 4-fluoro or 2,4-difluoro substitution.

7. A pharmaceutical composition comprising, in a pharmaceutically acceptable carrier, a compound according to any previous claim.

8. A compound according to any of claims 1-6 for use in medicine.

9. The use of a compound according to any of claims 1-6 for the manufacture of a medicament for treating or preventing a condition selected from: sleep disorders, schizophrenia, depression, anxiety, panic disorders, obsessive-compulsive disorder, pain, eating disorders, dependency or acute toxicity associated with narcotic agents: or for controlling extrapyramidal symptom associated with administration of neuroleptic agents; or for lowering intraocular pressure; or for treating hot flashes associated with the menopause.

## Patentansprüche

1. Eine Verbindung der Formel I: 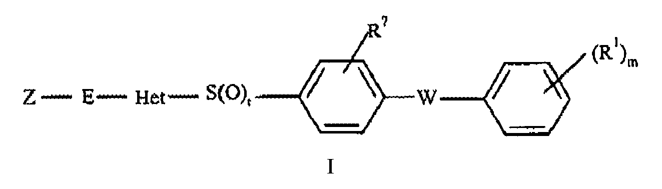 oder ein pharmazeutisch annehmbares Salz davon, wobei:
m 0, 1, 2 oder 3 ist,
t 1 oder 2 ist,
Het einen Pyridin- oder Thiophenring bedeutet, der 0, 1 oder 2 R²-Substituenten trägt,
W -CR³R⁴-CR⁵R⁶, -CR³=CR⁵- oder -C≡C- bedeutet, wobei R³, R⁴, R⁵ und R⁶ ausgewählt sind aus H, OH und F, wobei jedoch nicht mehr als einer der Reste R³, R⁴, R⁵ und R⁶ anders als H ist, oder R³ und R⁴ zusammen oder R⁵ und R⁶ zusammen eine Ketogruppe vervollständigen, oder R⁴ und R⁶ zusammen einen Cyclopropylring vervollständigen,
E eine chemische Bindung oder eine gerade oder verzweigte Alkylenkette bedeutet, die 1 bis 4 Kohlenstoffatome enthält, gegebenenfalls enthaltend ein Sauerstoffatom, um eine Etherverknüpfung zu bilden,
Z ausgewählt ist aus Halogen, CN, Nitro, CF₃, OCF₃, -R^{a}, -OR^{a}, -SR^{a}, -SOR^{a}, -SO₂R^{a}, -SO₂NR^{a}R^{b}, -NR^{a}R^{b}, -NR^{a}COR^{b}, -NR^{a}CO₂R^{b}, -NR^{a}CO₂NR^{a}R_{b}, -NR^{a}S(O)ₜR^{a}, -NR^{a}SO₂NR^{a}R_{b}, -COR^{a}, -CO₂R^{a}, -CONR^{a}R^{b}, -CH=NOR^{a} oder einem fünf- oder sechsgliedrigen heteroaromatischen Ring, der gegebenenfalls bis zu 2 Substituenten trägt, ausgewählt aus Halogen, CN, CF₃, C₁₋₆-Alkyl, C₁₋₆-Alkoxy, C₁₋₆Alkylthio, Amino, C₁₋₆-Alkylamino und Di(C₁₋₆)alkylamino,
R^{a} und R^{b} unabhängig H oder eine Kohlenwasserstoffgruppe mit bis zu 7 Kohlenstoffatomen bedeutet, die gegebenenfalls substituiert ist mit bis zu 3 Fluoratomen und gegebenenfalls mit Cl, Br, CN, OH, C₁₋₄-Alkoxy, C₁₋₄-Alkylthio, Amino, C₁₋₄-Alkylamino oder Di(C₁₋₄)alkylamino; oder R^{a} und R^{b}, wenn sie durch ein Stickstoffatom verknüpft sind, zusammen den Rest eines heterocyclischen Rings aus 4, 5 oder 6 Gliedern darstellen, gegebenenfalls bis zu 3 Substituenten tragend, ausgewählt aus Halogen, CN, CF₃, Oxo, OH, C₁₋₄-Alkyl und C₁₋₄-Alkoxy,
jedes R¹ unabhängig Halogen, CN, CF₃, OCF₃, C₁₋₆-Alkyl, OH, Benzylthio, C₁₋₆-Alkoxy oder Hydroxymethyl bedeutet,
jedes R² unabhängig Halogen, CN, CONH₂, C₁₋₄-Alkyl oder C₁₋₄-Alkoxy bedeutet
und R⁷ H, Halogen, CN, CF₃, OR^{a}, CO₂R^{a}, CONR^{a}R^{b}, NR^{a}R^{b} oder C₁₋₄-Alkyl bedeutet, das gegebenenfalls substituiert ist mit Halogen, CN, CF₃, OR^{a}, CO₂R^{a}, CONR^{a}R^{b} oder NR^{a}R^{b}.

2. Eine Verbindung gemäß Anspruch 1 der Formel II oder Formel III: 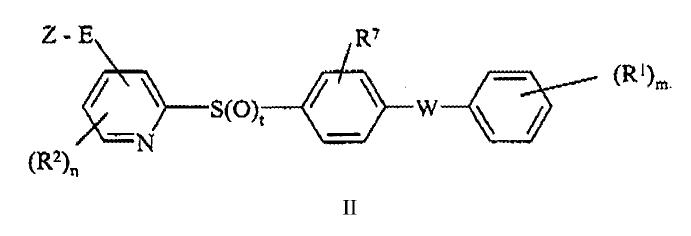 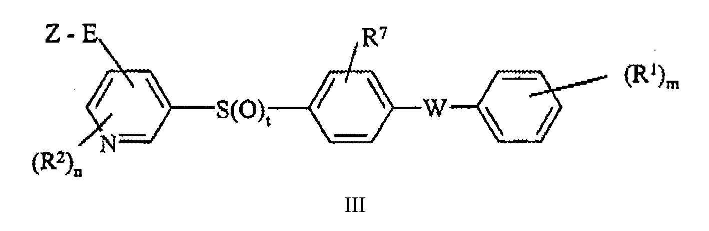 oder ein pharmazeutisch annehmbares Salz davon,
wobei n 0, 1 oder 2 ist und die anderen Variablen wie in Anspruch 1 definiert sind.

3. Eine Verbindung gemäß Anspruch 2, wobei der Rest Z-E- an eine Ringposition geknüpft ist, die zum Verknüpfungspunkt des -S(O)ₜ-Rests benachbart ist.

4. Eine Verbindung gemäß Anspruch 2, wobei der Rest Z-E- an eine Ringposition geknüpft ist, die zum Ringstickstoff benachbart ist.

5. Eine Verbindung gemäß einem vorhergehenden Anspruch, wobei Z-E- ausgewählt ist aus H, Isopropyl, 2-Cyanoethyl, Hydroxymethyl, 1-Hydroxyethyl, 2-Hydroxyethyl, 1-Hydroxypropyl, 1-Hydroxy-1-methylethyl, 1-Hydroxy-2,2,2-trifluorethyl, 1-Hydroxycyclobutyl, CO₂Me, CO₂Et, CONH₂, CONHMe, COCH₃, NH₂, NHMe, NMe₂, NHSO₂Me, SO₂Me, CN, CH₂NH₂, CH₂NHSO^{t}Bu, CH₂NHCOMe, Morpholin-4-yl und Morpholin-4-ylmethyl.

6. Eine Verbindung gemäß einem vorhergehenden Anspruch, wobei (R¹)ₘ eine 4-Fluor- oder 2,4-Difluor-Substitution bedeutet.

7. Eine pharmazeutische Zusammensetzung, die eine Verbindung gemäß einem vorhergehenden Anspruch in einem pharmazeutisch annehmbaren Träger umfasst.

8. Eine Verbindung gemäß einem der Ansprüche 1 - 6 zur Verwendung in der Medizin.

9. Die Verwendung einer Verbindung gemäß einem der Ansprüche 1 - 6 zur Herstellung eines Medikaments zur Behandlung oder Prävention eines Zustandes, ausgewählt aus: Schlafstörungen, Schizophrenie, Depression, Angst, Panikstörungen, obsessiv-kompulsive Störung, Schmerz, Essstörungen, Abhängigkeit oder akute Toxizität in Verbindung mit Narkotika, oder zur Steuerung extrapyramidaler Symptome in Verbindung mit der Verabreichung von Neuroleptika, oder zur Verringerung des Augeninnendrucks, oder zur Behandlung von Hitzewallungen in Verbindung mit der Menopause.

## Revendications

1. Composé de la formule I: 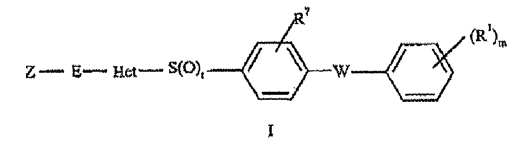 ou sel pharmaceutiquement acceptable de celui-ci; dans lequel:
m est 0, 1, 2 ou 3;
test 1 ou 2;
Het représente un cycle pyridine ou thiophène portant 0, 1 ou 2 substituants R²;
W représente -CR³R⁴-CR⁵R⁶, -CR³=CR⁵- ou -C≡C-, où R³, R⁴, R⁵ et R⁶ sont choisis parmi H, OH et F mais pas plus d'un de R³, R⁴, R⁵ et R⁶ est autre que H; ou R³ et R⁴ ensemble ou R⁵ et R⁶ ensemble complètent un groupe céto; ou R⁴ et R⁶ ensemble complètent un cycle cyclopropyle;
E représente une liaison chimique ou une chaîne alkylène droite ou ramifiée contenant de 1 à 4 atomes de carbone, incorporant optionnellement un atome d'oxygène pour former une liaison éther;
Z est choisi parmi un halogène, un groupe CN, nitro, CF₃, OCF₃, -R^{a}, -OR^{a}, -SR^{a}, -SOR^{a}, -SO₂R^{a}, -SO₂NR^{a}R^{b}, -NR^{a}R^{b}, -NR^{a}COR^{b}, -NR^{a}CO₂R^{b}, -NR^{a}CO₂NR^{a}R^{b}, -NR^{a}S(O)ₜR^{a}, -NR^{a}SO₂NR^{a}R^{b}, -COR^{a}, -CO₂R^{a}, -CONR^{a}R^{b}, -CH=NOR^{a} ou un cycle hétéroaromatique à cinq ou six membres portant optionnellement jusqu'à 2 substituants choisis parmi un halogène, des groupes CN, CF₃, alkyle Ci-6, alcoxy C₁₋₆, alkylthio C₁₋₆, amino, alkylamino C₁₋₆ et dialkyl(C₁₋₆)amino;
R^{a} et R^{b} représentent indépendamment H ou un groupe hydrocarbure de jusqu'à 7 atomes de carbone qui est optionnellement substitué avec jusqu'à 3 atomes de fluor et optionnellement avec Cl, Br, un groupe CN, OH, alcoxy C₁₋₄, alkylthio C₁₋₄, amino, alkylamino C₁₋₄ ou dialkyl(C₁₋₄)amino; ou R^{a} et R^{b}, lorsqu'ils sont liés par un atome d'azote, représentent ensemble le résidu d'un cycle hétérocyclique de 4, 5 ou 6 membres, portant optionnellement jusqu'à 3 substituants choisis parmi un halogène, des groupes CN, CF₃, oxo, OH, alkyle C₁₋₄ et alcoxy C₁₋₄;
chaque R¹ représente indépendamment un halogène, un groupe CN, CF₃, OCF₃, alkyle C₁₋₆, OH, benzylthio, alcoxy C₁₋₆ ou hydroxyméthyle;
chaque R² représente indépendamment un halogène, un groupe CN, CONH₂, alkyle C₁₋₄ ou alcoxy C₁₋₄;
et R⁷ représente H, un halogène, un groupe CN, CF₃, OR^{a}, CO₂R^{a}, CONR^{a}R^{b}, NR^{a}R^{b} ou alkyle C₁₋₄ qui est optionnellement substitué avec un halogène, un groupe CN, CF₃, OR^{a}, COR^{a}, CONR^{a}R^{b} ou NR^{a}R^{b}.

2. Composé selon la revendication 1 de la formule II ou de la formule III: 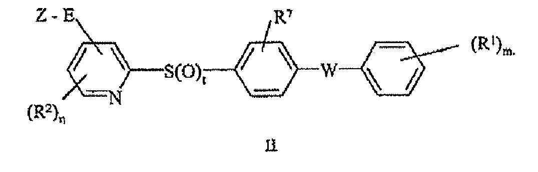 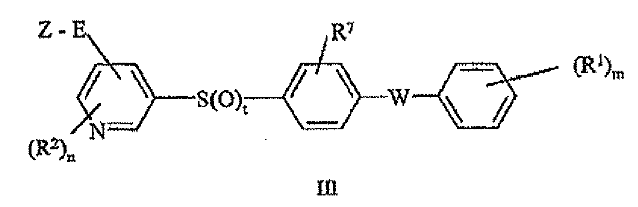 ou sel pharmaceutiquement acceptable de celui-ci;
dans lequel n est 0, 1 ou 2 et les autres variables sont telles que définies dans la revendication 1.

3. Composé selon la revendication 2, dans lequel la fraction Z-E- est attachée à une position de cycle qui est adjacente au point d'attache de la fraction -S(O)ₜ-.

4. Composé selon la revendication 2, dans lequel la fraction Z-E- est attachée à une position de cycle adjacente à l'azote du cycle.

5. Composé selon l'une quelconque des revendications précédentes, dans lequel la fraction Z-E- est choisie parmi H, des groupes isopropyle, 2-cyanoéthyle, hydroxyméthyle, 1-hydroxyéthyle, 2-hydroxyéthyle, 1-hydroxypropyle, 1-hydroxy-1-méthyléthyle, 1-hydroxy-2,2,2-trifluoroéthyle, 1-hydroxycyclobutyle, CO₂Me, CO₂Et, CONH₂, CONH₂, CONHMe, COCH₃, NH₂, NHMe, NMe₂, NHSO₂Me, SO₂Me, CN, CH₂NH₂, CH₂NHSO^{t}Bu, CH₂NHCOMe, morpholin-4-yle et morpholin-4-ylméthyle.

6. Composé selon l'une quelconque des revendications précédentes, dans lequel (R¹)ₘ représente une substitution 4-fluoro ou 2,4-difluoro.

7. Composition pharmaceutique comprenant, dans un support pharmaceutiquement acceptable, un composé selon l'une quelconque des revendications précédentes.

8. Composé selon l'une quelconque des revendications 1-6 pour une utilisation en médecine.

9. Utilisation d'un composé selon l'une quelconque des revendications 1-6 pour la fabrication d'un médicament pour le traitement ou la prévention d'un état choisi parmi: des troubles du sommeil, une schizophrénie, une dépression, une anxiété, des troubles de panique, un trouble obsessionnel compulsif, une douleur, des troubles de l'alimentation, une dépendance ou une toxicité aiguë associée à des agents narcotiques; ou pour le contrôle de symptômes extrapyramidaux associés à l'administration d'agents neuroleptiques; ou pour l'abaissement de la pression intraoculaire; ou pour le traitement de bouffées de chaleur associées à la ménopause.
